(19) **Europäisches Patentamt**
**European Patent Office**
**Office européen des brevets**

(11) **EP 1 997 888 A1**

(12) **EUROPEAN PATENT APPLICATION**
published in accordance with Art. 158(3) EPC

(43) Date of publication:
**03.12.2008 Bulletin 2008/49**

(51) Int Cl.:
*C12N 15/09* (2006.01)   *C12M 1/00* (2006.01)
*C12Q 1/68* (2006.01)   *G01N 27/28* (2006.01)
*G01N 27/327* (2006.01)   *G01N 35/08* (2006.01)
*G01N 37/00* (2006.01)

(21) Application number: **07739538.2**

(22) Date of filing: **23.03.2007**

(86) International application number:
**PCT/JP2007/056097**

(87) International publication number:
**WO 2007/111274 (04.10.2007 Gazette 2007/40)**

(84) Designated Contracting States:
**DE FR GB**

(30) Priority: **24.03.2006 JP 2006083293**

(71) Applicant: **Kabushiki Kaisha Toshiba**
**Minato-ku,**
**Tokyo 105-8001 (JP)**

(72) Inventors:
• **HONGO, Sadato**
  **2 (JP)**
• **OKADA, Jun**

  **(JP)**

• **OOKI, Kenji**

  **(JP)**
• **GEMMA, Nobuhiro**

  **(JP)**

(74) Representative: **Lord, Hilton David**
**Marks & Clerk**
**90 Long Acre**
**London**
**WC2E 9RA (GB)**

(54) **NUCLEIC ACID DETECTION CASSETTE AND NUCLEIC ACID DETECTION APPARATUS**

(57)    A nucleic acid cassette includes a main body in which first and second channels are formed. A sample containing a target nucleic acid is input in a sample chamber of the second channel via an injection port. In the cassette, a detecting part is incorporated which includes a detection chamber communicated to the first and second channels and a nucleic detection unit. The first channel has a first reagent reservoir storing a first reagent stored and a second reagent reservoir storing a second reagent. The first and second reagents reservoirs are separated by a gaseous body in the channel. The sample and the first and second reagents are supplied to the detecting part due to an action of a pump part which is communicated to the first and second channels. Accordingly, it is possible to prevent a non-detection target nucleic monocle from being mixed and a nucleic acid sample from being leaked to the outside.

FIG.1

EP 1 997 888 A1

SEQUENCE LISTING

<110> KABUSHIKI KAISHA TOSHIBA

<120> NUCLEIC ACID DETECTION CASSETTE AND NUCLEIC ACID DETECTION DEVICE

<130> 06S1134P

<140> PCT/JP2007/056097
<141> 2007-03-23

<150> JP 2006-083293
<151> 2006-03-24

<160> 10

<170> PatentIn version 3.3

<210> 1
<211> 15
<212> DNA
<213> Probe

<400> 1
atgctttccg tggca                                           15


<210> 2
<211> 15
<212> DNA
<213> Probe

<400> 2
atgctttgcg tggca                                           15


<210> 3
<211> 15
<212> DNA
<213> Probe

<400> 3
atgctttccg tggca                                           15


<210> 4
<211> 15
<212> DNA
<213> Probe

<400> 4
atgctttgcg tggca                                           15


<210> 5
<211> 16

```
<212>  DNA
<213>  Human

<400>  5
ctgccacgga aagcat                                                  16


<210>  6
<211>  15
<212>  DNA
<213>  Probe

<400>  6
atgctttccg tggca                                                   15


<210>  7
<211>  15
<212>  DNA
<213>  Probe

<400>  7
atgctttgcg tggca                                                   15


<210>  8
<211>  15
<212>  DNA
<213>  Probe

<400>  8
atgctttccg tggca                                                   15


<210>  9
<211>  15
<212>  DNA
<213>  Probe

<400>  9
atgctttgcg tggca                                                   15


<210>  10
<211>  16
<212>  DNA
<213>  Human

<400>  10
ctgccacgga aagcat                                                  16
```

**Description**

Technical Field

The present invention relates to a nucleic acid detection cassette and a nucleic acid detection system for detecting a target nucleic acid to specify a base sequence, and particularly, to a nucleic acid detection cassette for full automatically processing a step for detecting a nucleic acid or a pretreatment step of detection of a nucleic acid in order to detect a target nucleic acid, and a nucleic acid detection system using the nucleic acid detection cassette.

Background Art

In accordance with development of a gene technology in recent years, diagnosis of disease based on a gene or preservation of disease is becoming possible in a medical field. Such a diagnosis is referred to as a genetic diagnosis. The genetic diagnosis enables diagnosis or prediction of disease before outbreak of the disease or on a very initial stage by detecting a defect or change of a gene of a human being which is a cause of the disease. In addition, a research associated with a relation between a genotype and an epidemic has been progressed along with decoding of a human genome, and treatment in response to an individual genotype (tailored medication) is being realized. Accordingly, it becomes very important to detect a gene simply and determine a genotype.

Conventionally, a nucleic acid detection system of a full automatic type for automatically carrying out from extraction of a nucleic acid up to data analysis has been developed. For example, JP-A 3-7571 (KOKAI) discloses a nucleic acid detection system capable of automatically processing amplification of a nucleic acid and detection of a nucleic acid. However, the system involves a problem such that a reliable countermeasure is not taken against incorporation of a non-target nucleic acid molecule which is not a target of detection, namely, so-called contamination. Also, the conventional nucleic acid detection system disclosed in the JP-A 3-7571 (KOKAI) is a system of a large size and it has a use application for research. A cassette for solving a problem of contamination is proposed in JP-A 2005-261298 (KOKAI). A nucleic acid detection system of a full automatic type disclosed in the JP-A 2005-261298 (KOKAI) attempts to solve a problem by adopting a closed type cassette against contamination. However, the cassette to be loaded in the system needs a plurality of valve functions and flow channels and this involves a problem such that the structure is complicated so as to prevent downsizing and simplification of the cassette and downsizing and simplification of the system itself.

Other than this, nucleic acid detection cassettes are also disclosed in USP No. 6,830,936 assigned to Affymetrix Inc., USP No. 6,605,454 assigned to Motorola and the like. The cassette disclosed in any of USP Nos. 6,830,936 and 6,605,454 has a channel formed therein and further, a valve mechanism mounted thereon, so that the structure of the cassette becomes complicated. The structure of the cassette becomes complicated, which may lead not only to lowering of reliability of the nucleic acid detection cassette but also to lowering of a sample amount or a sample density due to absorption in the channel, valve part or the like, contamination due to liquid leakage, loss of a detection time and others.

As described above, as a critical issue in development of the full automatic nucleic acid detection system, it is important to prevent contamination of a non-detection target nucleic acid molecule (a non-target nucleic acid molecule) and leakage of a nucleic acid sample to the outside. In view of such a background, there has been a desire not only to prevent contamination but also to develop a nucleic acid detection cassette having a small and simple structure and a full automatic nucleic acid detection system.

Disclosure of Invention

The present invention has been made taking the foregoing problems into consideration, and an object of the invention is to provide a nucleic acid detection cassette having a structure so as to prevent contamination of a non-detection target nucleic acid molecule and leakage of a nucleic acid sample to the outside and a nucleic acid detection system.

According to an aspect of the present invention, there is provided a nucleic acid detection cassette, comprising:

a first injection port which allows a sample containing a target nucleic acid to be injected therethrough;
a first channel;
a second channel which is communicated to the first channel and the injection port;
a detecting part comprising a detection chamber which is communicated with the first and second channels and is so formed as to be separated from the outside of the nucleic acid detection cassette, and a nucleic acid detection unit which detects an electric signal to determine the target nucleic acid in the sample supplied to the detection chamber, the detecting part being arranged between the first and second channels;
a first reagent reservoir which is defined in the first channel and stores a first reagent while separating the first reagent from the sample via a gaseous body;

a second reagent reservoir which is defined in the first channel and stores a second reagent while separating the second reagent from the first reagent and the sample via a gaseous body;

a sample chamber which is defined in the second channel and is communicated to the injection port, the sample chamber receiving the injected sample; and

a pump part which is arranged between the first and second channels so as to be communicated to the first and second channels, the pump part forming a closed path together with the first channel, the second channel, and the detecting part, a pressure being applied to the pump part from the outside of the nucleic acid detection cassette so that the sample and the first and second reagents are individually supplied to the detecting part.

According to another aspect of the present invention, there is provided a nucleic acid detection cassette, comprising:

a first injection port which allows a sample containing a target nucleic acid to be injected therethrough;

a first channel;

a second channel which is communicated to the first channel and the injection port;

a detecting part comprising a detection chamber which is communicated to the first and second channels and is so formed as to be separated from the outside of the nucleic acid detection cassette, and a nucleic acid detection unit which determines the target nucleic acid in the sample supplied to the detection chamber, the detecting part being arranged between the first and second channels;

a first reagent reservoir which is defined in the first channel and stores a first reagent while separating the first reagent from the sample via a gaseous body;

a sample chamber which is defined in the second channel and is communicated to the injection port, the sample chamber receiving the injected sample;

a sample storage chamber which is defined in the second channel, comprises a first channel port communicated to the sample chamber and a second channel port communicated to the detecting part, and stores a sample liquid containing the sample supplied from the sample chamber, the first and second channel ports being opened in the sample storage chamber above a liquid level of the sample liquid; and

a pump part which is arranged between the first and second channels so as to be communicated to the first and second channels, the pump part forming a closed path together with the first channel, the second channel, and the detecting part, a pressure being applied to the pump part from the outside of the nucleic acid detection cassette so that the sample and the first and second reagents are individually supplied to the detecting part.

According to still another aspect of the present invention, there is provided a nucleic acid detection cassette, comprising:

an injection port which allows a sample containing a target nucleic acid to be injected therethrough;

a first channel;

a second channel which is communicated to the first channel and the injection port;

a detecting part comprising a detection chamber which is communicated to the first and second channels and is so formed as to be separated from the outside of the nucleic acid detection cassette, and a nucleic acid detection unit which detects an electric signal to determine the target nucleic acid with the sample supplied to the detection chamber, the detecting part being arranged between the first and second channels;

a first reagent reservoir which is defined in the first channel and stores a first reagent while separating the first reagent from the sample via a gaseous body;

a second reagent reservoir which is defined in the first channel and stores a second reagent while separating the second reagent from the first reagent and the sample via a gaseous body;

a sample chamber which is defined in the second channel and is communicated to the injection port, the sample chamber receiving the injected sample;

a fourth reagent reservoir which is communicated to the sample chamber and stores a fourth reagent to be supplied to the sample chamber;

a sample storage chamber to store a sample liquid which corresponds to a mixture of the sample and the fourth reagent agent, the mixture being supplied from the sample chamber after processing, the sample storage chamber being defined in the second channel and comprising a first channel port communicated to the sample chamber and a second channel port communicated to the detecting part;

a first pump part provided on the cassette so as to be communicated to the second channel, the first pump part being actuated from the outside of the nucleic acid detection cassette so that the fourth reagent is supplied to the sample chamber; and

a second pump part which is arranged between the first and second channels so as to be communicated to the first and second channels, the second pump part forming a closed path together with the first and second channels and the detection part, a pressure being applied from the outside of the nucleic acid detection cassette to so that the mixture of the sample and the fourth reagent and the first and second reagents individually are supplied to the detection part.

Brief Description of Drawings

FIG. 1 is a block diagram schematically showing a nucleic acid detection cassette according to an embodiment of the invention.

FIG. 2A is a conceptual diagram schematically showing the operation of feeding a sample and a reagent to the nucleic acid detection cassette shown in FIG. 1.

FIG. 2B is a conceptual diagram schematically showing the operation of feeding the sample and the reagent to the nucleic acid detection cassette shown in FIG. 1.

FIG. 2C is a conceptual diagram schematically showing the operation of feeding the sample and the reagent to the nucleic acid detection cassette shown in FIG. 1.

FIG. 3 is a block diagram schematically showing a nucleic acid detection cassette having a plurality of sample injection sections according to another embodiment of the invention.

FIG. 4A is a conceptual diagram schematically showing arrangement in a plane, which is equivalent to a horizontal plane in the nucleic acid detection cassette shown in FIG. 1.

FIG. 4B is a conceptual diagram schematically showing arrangement in a cross section, which is equivalent to a vertical plane in the nucleic acid detection cassette shown in FIG. 1.

FIG. 5 is a block diagram schematically showing a nucleic acid detection cassette according to a modification of the nucleic acid detection cassette shown in FIG. 3.

FIG. 6 is a conceptual diagram for explaining an example of the liquid feeding operation of the nucleic acid detection cassette shown in FIGS. 4A and 4B.

FIG. 7 is a conceptual diagram for explaining an example of the liquid feeding operation of a modification of the nucleic acid detection cassette shown in FIGS. 4A and 4B.

FIG. 8 is a conceptual diagram for explaining another example of the liquid feeding operation of the nucleic acid detection cassette shown in FIGS. 4A and 4B.

FIG. 9 is a conceptual diagram for explaining still another example of the liquid feeding operation of the nucleic acid detection cassette shown in FIGS. 4A and 4B.

FIG. 10A is a conceptual diagram for explaining further another example of the liquid feeding operation of the nucleic acid detection cassette shown in FIGS. 4A and 4B.

FIG. 10B is a conceptual diagram for explaining still another example of the liquid feeding operation of the nucleic acid detection cassette shown in FIGS. 4A and 4B.

FIG. 11A is a conceptual diagram for explaining still another example of the liquid feeding operation of the nucleic acid detection cassette shown in FIGS. 4A and 4B.

FIG. 11B is a conceptual diagram for explaining still another example of the liquid feeding operation of the nucleic acid detection cassette shown in FIGS. 4A and 4B.

FIG. 12 is a view schematically showing top, front, and sides of a nucleic acid detection cassette according to a specific example of the nucleic acid detection cassette shown in FIG. 3.

FIG. 13 is a perspective view schematically showing the nucleic acid detection cassette according to the specific example of the nucleic acid detection cassette shown in FIG. 3.

FIG. 14A is a top view schematically showing a nucleic acid extraction and amplification section shown in FIGS. 11A, 11B, 11C and 11D.

FIG. 14B is a side view schematically showing the nucleic acid extraction and amplification section shown in FIGS. 11A, 11B, 11C and 11D.

FIG. 15 is side view showing various sample-sampling bars to be introduced in a sample injection port of the nucleic acid extraction and amplification section shown in FIG. 12; and is bottom view showing various sample-sampling bars to be introduced in a sample injection port of the nucleic acid extraction and amplification section shown in FIG. 12.

FIG. 16 is a side view showing a sample chamber shown in FIG. 12 in detail.

FIG. 17 is side view showing another example of the sample chamber shown in FIG. 12 in detail; and is side view showing an example of a buffer section arranged behind the sample chamber shown in FIG. 12.

FIG. 18 is a block diagram schematically showing a nucleic acid detection system using the nucleic acid detection cassette according to the embodiment.
FIG. 19 is a flow chart showing the processing from injection of a sample up to detection of a nucleic acid performed in the nucleic acid detection system shown in FIG. 18.

Best Mode for Carrying Out the Invention

Hereinafter, with reference to the drawings, there will be described a nucleic acid detection cassette and a nucleic acid detection system according to an embodiment of the present invention.

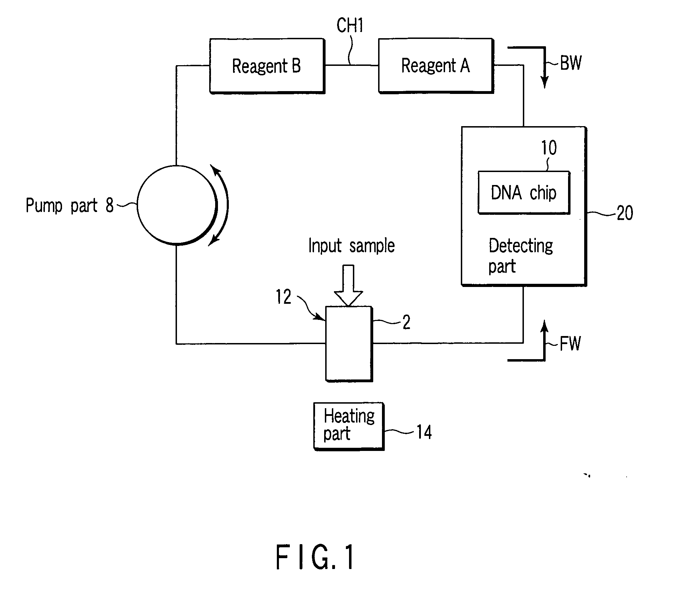

FIG. 1 schematically shows a structure of a nucleic acid detection cassette to be inserted in a nucleic acid detection system according to an embodiment of the present invention, the nucleic acid detection system applying a nucleic acid detection method of a current detection type using an intercalator.

As shown in this FIG. 1, the nucleic acid detection cassette is provided with annular channels closed in an annular manner (distribution channels closed in an annular manner) CH1 (first channel) and CH2 (second channel) (hereinafter, described as "channel CH1" and "channel CH2", respectively). In the channel CH1, a liquid-state sample 2 is received. A liquid-state reagent A (first reagent) such as a washing reagent (buffer solution) and a liquid-state reagent B (second reagent) such as an intercalator (a solution of a substance which is bound to a hybridization-reacted nucleic acid and makes an electro-chemical reaction) are also received in the channel CH1 in such a manner that the liquid-state sample 2, the liquid-state reagent A, and the liquid-state reagent B are spatially separated by gaseous body. Namely, the channel CH1 has a first reagent reservoir for storing the first reagent and a second reagent reservoir for storing the second reagent.

Also arranged is a pump part 8 for moving the sample 2, the reagent A, and the reagent B, which are in the form of liquid, through the channels CH1 and CH2 in communication with the channels CH1 and CH2, for example, a pump of a peristaltic system provided with a pressure roller and an elastic piping tube as to be described later.

As shown in FIG. 1, a detection chamber separated from the outside is communicated to the channels CH1 and CH2, and a DNA chip 10 (a nucleic acid detection unit) is arranged in the detection chamber, so that the detection chamber and the DNA chip 10 form a detecting part 20.

In addition, the channel CH2 is provided with a sample chamber 12 in which the sample 2 is injected. The sample chamber 12 has a sample injection port and is constituted so that the sample 2 can be injected from the outside in the channel CH2. The injection port of the sample chamber 12 is released to the sample 2 only when injecting the sample 2, and after the sample 2 is injected, the injection port of the sample chamber 12 is closed. The sample chamber 12 may be provided with a heating section 14, which enables to heat the sample 2 in the sample chamber 12.

The liquid-state reagent A and the liquid-state reagent B are arranged in the channel CH1 being partitioned each other by gaseous body. The sample 2 injected is also arranged in the channel CH2 being separated by gaseous body.

The pump part 8 is coupled to the detecting part 20 having the DNA chip 10 arranged via the liquid-state reagent A, the liquid-state reagent B, and gaseous body in the channel CH1. Also, the pump part 8 is coupled to the detecting part 20 having the DNA chip 10 arranged in the detection chamber via gaseous body in the channel CH2 and the sample 2. The pump part 8 supplies the sample 2 and the reagents A and B to the detecting part 20 by providing a pump channel in the main body of the cassette and operating the pump channel from the outside. The channels CH1 and CH2 are defined to be a closed structure in the main body of the nucleic acid detection cassette. The detecting part 20 provided with the DNA chip 10 and the detection chamber is incorporated so as to be sealed in the main body of the cassette in the same way. In other words, after closing the sample injection port, the channels CH1 and CH2, the pump part 8 arranged between the channels CH1 and CH2, and the detecting part 20 forms a closed channel closed to the outside.

In this specification, the description of the details of a nucleic acid detection method of a current detection type using an intercalator is omitted. With respect to the details of the nucleic acid detection method, please refer to USP 5,776,672 allowed a patent on July 7, 1998 and USP 5,972,692 allowed a patent on October 26, 1999 (both by Koji Hashimoto et. al and Assignee Kabushiki Kaisha Toshiba) as well as the corresponding Japanese Patent No. 2573443. The description of the specification of the USP should form a part of this specification.

The DNA chip 10 according to the present embodiment is a DNA chip of a current detection type using an intercalator. The DNA chip 10 has a plurality of individual electrodes arranged on an insulating substrate, and a probe DNA is immobilized onto each of the individual electrodes. It is preferable that the individual electrodes are juxtaposed in a matrix. In addition, a counter electrode is arranged as to face the individual electrode, and a reference electrode is also arranged on the DNA chip 10. The individual electrodes are made of Au or the like.

When the sample 2 (the DNA sample) is supplied to the DNA chip 10, a hybridization reaction is caused between a target DNA and a probe DNA contained in the sample 2. The DNA chip 10 is then washed by using a washing reagent. Thereafter, the intercalator is made to react to the hybridization-reacted nucleic acid. A voltage is applied between the counter electrode and the individual working electrode so that a current flows through the individual electrode due to an electro-chemical reaction of the intercalator. Detecting the current makes it possible to specify the existence of the target DNA.

A probe chamber to which the DNA sample is supplied is provided in the DNA chip 10 of the detecting part 20. The detection chamber is provided with ports coupled to the channels CH1 and CH2, and other than the port is separated from the outside.

The nucleic acid detection cassette shown in FIG. 1 is constituted so as to be sealed against the outside after the sample 2 is first injected from the outside in the sample chamber 12. It is preferable that, with a reagent for preprocessing (or extracting) and/or amplifying a nucleic acid stored in advance in the sample chamber 12, the sample is heated by the heating section 14 changed into a DNA sample obtained by extracting/amplifying the target DNA. The DNA sample may be injected as the sample 2 from the outside. In this case, the reagent stored in the sample chamber 12 in advance may be only a reagent for amplifying a nucleic acid.

Thereafter, the pump part 8 is operated in a forward direction FW in which the sample 2 is supplied to the DNA chip 10.

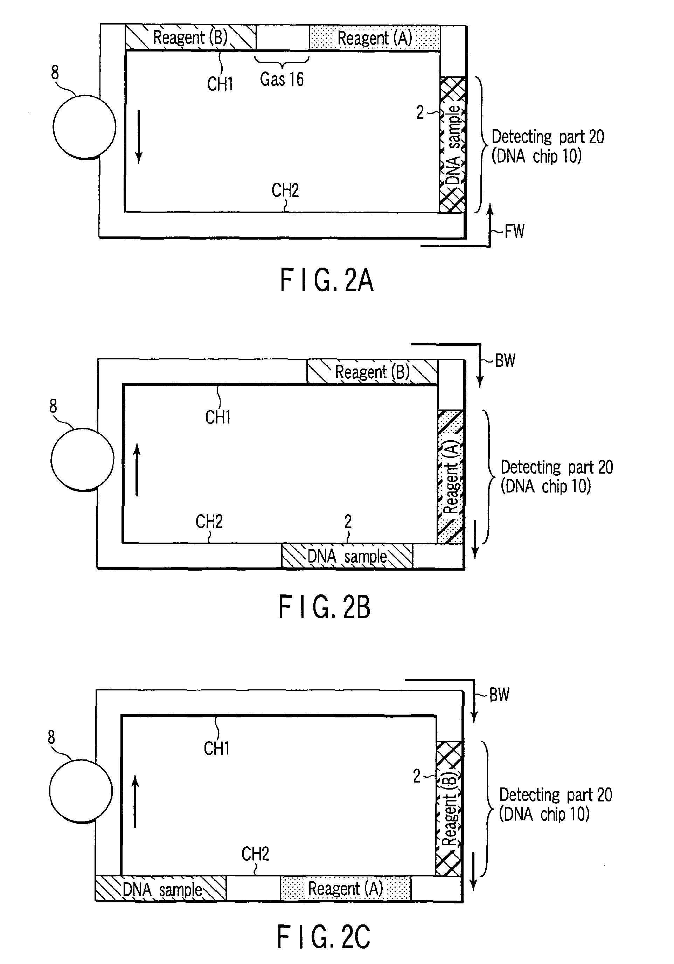

As shown in FIG. 2A, gaseous body in the channel CH2 is pressed by a pressing force from the pump part 8 to feed the sample 2 to the DNA chip 10, so that the target DNA is connected (hybridization-reacted) to the probe DNA of the DNA chip 10.

Next, the pump part 8 is operated in a backward direction BW, and the reagent A is pressed by gaseous body 16 and the reagent B in the channel CH1, so that the reagent A is supplied to the DNA chip 10 of the detecting part 20. As shown in FIG. 2B, most of the sample (DNA sample) 2 is pushed out to the channel CH2, outside of the DNA chip 10 by a pressure in response to inflow of the reagent A. In the DNA chip 10, along with supply of the reagent A to the DNA chip 10, DNA except for the target DNA coupled to the probe DNA of the DNA chip 10 and having a complementary sequence to the probe DNA is washed away by the reagent A. Further, if the pump part 8 continues to be operated in the backward direction BW, the reagent B further pushes gaseous body as shown in FIG. 2C, so that the reagent A containing DNA which is washed away from the DNA chip is pushed out to the channel CH2 outside the DNA chip 10.

If the pump part 8 continues to be operated, the reagent B is moved to the DNA chip 10 due to the pressure of gaseous body. In the DNA chip 10, a molecule of the intercalator is bound to a hybridized strand of the probe DNA on the DNA chip 10.

When a voltage is applied between the counter electrode and the individual working electrode, an oxidation current arising from the intercalator bound to the hybridized DNA is detected on the individual working electrode. Since the base sequence of the probe DNA of the DNA chip 10 is known, the base sequence of the target DNA can be specified by specifying the individual electrode having detected the oxidation-reduction current. In other words, it is turned out that the base sequence of the detection target area of the target DNA is complementary to the base sequence of the probe DNA of the current-detected electrode.

In the case where the DNA chip 10 is not a chip for the nucleic acid detection method of the current detection type using the intercalating agent, the reagent B (intercalating agent) is not always needed. As a result, the area (second reagent reservoir) receiving the reagent B in the channel CH1 of the nucleic acid detection cassette is not always needed.

The reagent A and reagent B are arranged in the order of "the reagent A - the gaseous body 16 - the reagent B" from the detection area of the DNA chip 10 in the channel CH1. Since there is no extremely wide part in the channel CH1, they move in parallel from their original positions by a pressure from the pump part 8, so that they maintain the order of "the reagent A - the gas 16 - the reagent B". Accordingly, when the reagent A is desired to be arranged in the detection area, the reagent A may be moved to the detection area by the pump. The reagent A is positioned in the detection area by controlling the feeding amount of the pump part 8. A liquid detection sensor can be arranged in a channel in the detecting part 20 or in the vicinity of the detecting part 20. In that case, if the sensor detects that the reagent A reaches the detection area, the pump part 8 may be controlled so as to stop its liquid feeding operation.

The reagents A and B are severed (partitioned) by the gas 16 and are prevented from being mixed with each other when feeding a liquid, and only the reagent A can be arranged in the detection area. Next, in the case of intending to move the reagent B to the detection area, the reagent B may be moved by the pump part 8 in the same way. Thereby, the reagent A is discharged from the detection area, and the reagents A and B are separated by the gas 16. For this reason, the reagents A and B are not mixed with each other, and the regent B is arranged on the detection area.

When the severe discussion is given, the remaining reagent attached to the channel or the surface of the detection area and the reagent to be fed afterward are slightly mixed. However, since the gaseous body 16 is arranged between the reagent A and the reagent B, most of the former reagent A is discharged by the gaseous body 16, which makes it possible to suppress mixture of the reagents to the minimum. In this way, the reagent A and the reagent B are separated by the gaseous body 16 to be fed, and thus, the reagent A and the reagent B can be switched to be supplied. In addition, it is not necessary to arrange a valve in the reservoirs for the reagent A and the reagent B, and as a result, downsizing of the detection cassette can be realized.

The closed channels CH1 and CH2 as shown in FIGS. 2A to 2C do not necessarily have equal cross sections and they have some channel portions having relatively large sections and other channel portions having relatively small sections. In some portions of the channels CH1 and CH2 having smaller sections than other portions of the channels CH1 and CH2 having larger sections depending on shapes of the channels CH1 and CH2, wet properties of the surfaces of the channels CH1 and CH2, surface tensions of the sample 2 and the reagents A and B as liquids, viscosities of the sample 2 and the reagents A and B as liquids, and volumes of the sample 2 and the reagents A and B as liquids, the sample 2 and the reagents A and B as liquids always cover the sections of the reagents A and B.

It is preferable that the channels CH1 and CH2 each have an area where a direction of the channel is substantially horizontal in a gravity direction and an area where the direction of the channel is substantially vertical in the gravity direction when mounting the nucleic acid detection cassette on a substantially horizontal plane. All the reagents A and B and the sample are arranged and formed on the former area. This enables to stably store a liquid. In this case, it is preferable that the channel section of the area where the direction of the channel is substantially vertical in the gravity direction is smaller than the area where the direction of the channel is substantially horizontal in the gravity direction in order to stably store a liquid in each reagent reservoir.

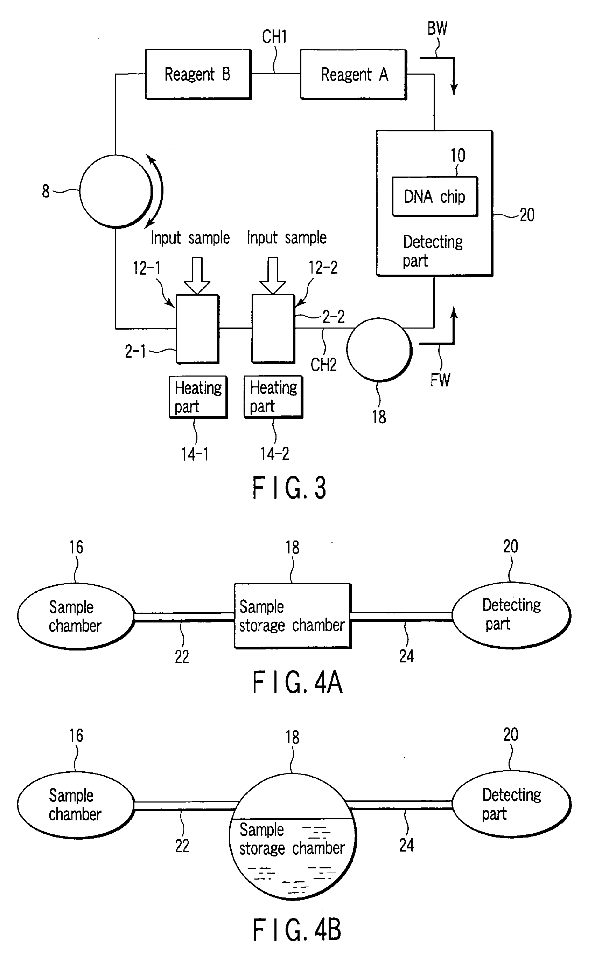

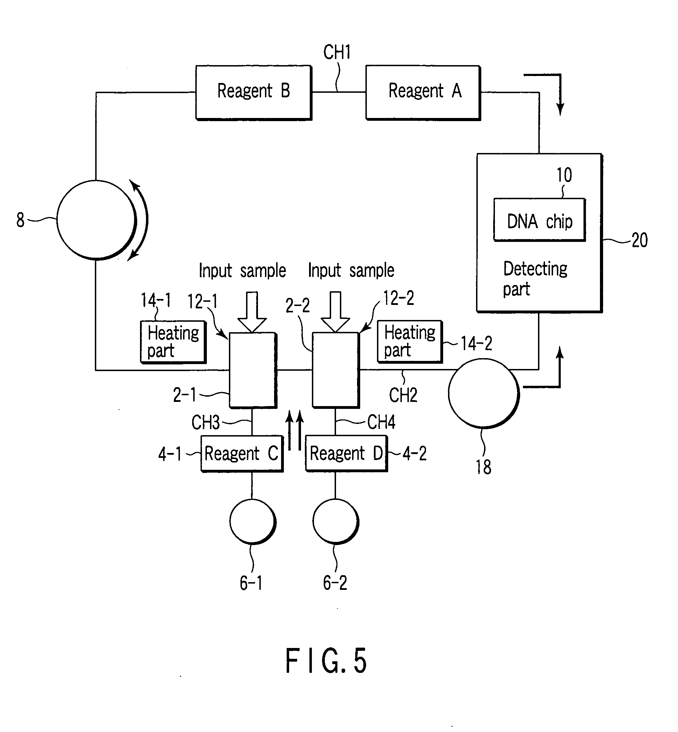

The channel shown in FIG. 1 is closed. However, if a closed channel is configured to thereby individually supply the liquid-state sample 2, the liquid-state reagent A such as a washing reagent (buffer solution) and the liquid-state reagent B in this channel, it is obvious that the channel is not limited to an annular channel. FIG. 3 is a block diagram showing a nucleic acid detection cassette according to another embodiment. FIG. 4A is a plan view showing a partial planar alignment of the nucleic acid detection cassette shown in FIG. 3, and FIG. 4B is a plan view showing a sectional alignment of a part of the nucleic acid detection cassette shown in FIG. 3. FIG. 5 is a block diagram showing a nucleic acid detection cassette according to a modification of the nucleic acid detection cassette shown in FIG. 3.

In FIGS. 3 and 5, a place or a part given the same reference marks as those shown in FIG. 1 shows the same place or the same part shown in FIG. 1, and its explanation is omitted.

In the cassette shown in FIG. 3, the channel CH2 is provided with first and second sample chambers 12-1 and 12-2 having sample injection ports that allow first and second samples 2-1 and 2-2 to be injected therethrough, respectively. Accordingly, the first and second samples 2-1 and 2-2 can be supplied to the DNA chip 10 via the first and second sample chambers 12-1 and 12-2, respectively. The first and second sample chambers 12-1 and 12-2 respectively have first and second heating ports 14-1 and 14-2 for heating the samples 2-1 and 202, and it is possible to heat the samples 2-1 and 2-2 at a temperature in response to the samples 2-1 and 2-2. According to need, two or more sample chambers may be provided.

In the cassette shown in FIG. 3, a sample storage chamber 18 for mixing the samples 2-1 and 2-2 is arranged between the second sample 12-2 and the DNA chip 10. The sample storage chamber 18 is provided in the channel CH2 in order to mix the samples 2-1 and 2-2. However, in the case of supplying the reagent obtained by mixing the reagents A and B to the DNA chip 10, the sample storage chamber 18 may be provided in the channel CH1. Further, the sample storage chamber 18 may store in advance various reagents (third reagent) for controlling a salt concentration for a hybridization reaction on the DNA chip or for detecting a nucleic acid, so that it is also possible to mix the reagent with the sample by feeding the samples 2-1 and 2-2 to the sample storage chamber.

Here, the sample storage chamber 18 has a input port (first channel port) which is communicated to the second sample 12-2 and an output port (second channel port) which is communicated to the detecting part 20. With the main body of the cassette kept horizontally, the reagent is stored such an amount that the reagent stored in the sample storage chamber 18 is not leaked to the outside of the sample storage chamber 18 via the input port or the output port. In other words, a liquid level of the stored reagent is determined so as to be lower than the input port or the output port. Further, also in a state that the samples 2-2 and 2-1 are added to the reagent stored in the sample storage chamber 18, the capacity of the sample storage chamber or the amount of the reagent, and the amount of the sample is determined such that the reagent stored in the sample storage chamber 18 is not leaked to the outside of the sample storage chamber 18 via the input port and the output port if the main body of the cassette is substantially horizontally kept. In addition, for one sample chamber 12, an auxiliary sample chamber communicated to the sample chamber 12 may be arranged in the channel CH2.

As shown in FIG. 5, auxiliary channels CH3 and CH4, which are different from the channel CH2, are coupled to the first and second samples 12-1 and 12-2 having the heating ports 14-1 and 14-2, respectively. Reagent holding areas 4-1 and 4-2 are coupled to the auxiliary channels CH3 and CH4, respectively. For example, reagents C and D (fourth reagent) for amplifying a nucleic acid are charged in these reagent holding areas 4-1 and 4-2, and extruding sections 6-1 and 6-2 are communicated to the reagent holding areas 4-1 and 4-2, respectively. When the extruding sections 6-1 and 6-2 are operated in the nucleic acid detection cassette shown in FIG. 5, a pressure arising from deformation of the extruding sections 6-1 and 6-2 allows the reagents C and D within the reagent holding areas 4-1 and 4-2 to be supplied to the first and second sample chambers 12-1 and 12-2, to which the reagents C and D correspond, respectively. In the nucleic acid detection cassette shown in FIG. 5, after injecting the samples 2-1 and 2-2 in the first and second sample chambers 12-1 and 12-2 and heat-treating them, the reagents C and D can be injected in the sample chambers 12-1 and 12-2. In other words, temperature management of the sample chamber before and after inputting the reagents C and D can be individually realized. As a consequence, the nucleic acid detection cassette shown in FIG. 5 is constituted in such a manner that, when boil-treating a test body and extracting a nucleic acid, an enzyme with no heat resistance is evacuated from a heat-treatment section, and an enzyme is added to carry out a nucleic acid amplification reaction after boil-treatment is terminated, thereby effectively carrying out the reaction. In this way, it is possible to set various reaction conditions suitable for respective reagents.

According to the cassette shown in FIG. 1, an example that a single sample chamber 12 is arranged is shown. Further, according to the cassette shown in FIG. 3, there is shown an example that the first and second sample chambers 12-1 and 12-2 and the sample storage chamber 18 are arranged. However, the sample storage chamber 18 may be provided to the channel CH2 shown in FIG. 1 in addition to the sample chamber 12, and the sample 2 and the reagent may be mixed in the sample storage chamber 18.

In any case of FIGS. 1, 3 and 5, with for example, a reagent for extracting/amplifying a nucleic acid stored in advance in the sample chamber 12, the sample injected, thereby enabling to process the sample in the reagent. If there is a plurality of sample chambers as shown in FIGS. 3 and 5, the reagent to be stored in advance may be different for each chamber or may be the same. In addition, the reagent for extracting/amplifying the nucleic acid may be stored in the sample chambers 12, 12-1 and 12-2. Alternatively, the reagent for extracting the nucleic acid may be stored in the sample chambers 12, 12-1 and 12-2, while the reagent for amplifying the nucleic acid may be stored in the first and second holding areas 14-1 and 14-2, separately.

As shown in FIG. 4A, the sample storage chamber 18 is arranged between the sample chamber 16 as the sample chambers 12-1 and 12-2 and the detecting part 20 having the DNA chip 10 arranged thereon. When viewed from a three-dimensional section, the sample storage chamber 18 is preferably arranged in a positional relation shown in FIG. 4B as to be described later.

As explained with reference to FIG. 3, for detecting the nucleic acid, it is necessary to mix the sample 2-1 with the sample 2-2 and further, it is necessary to mix the samples 2-1 and 2-2 to the reagent held in the sample storage chamber. For this mixture, as typically shown in FIGS. 3, 4A and 4B, the sample storage chamber 18 is arranged between the sample chamber 16 in the sample chamber 12 and the detecting part 20 having the DNA chip 10.

In the nucleic acid detection by means of the nucleic acid detection cassette shown in FIGS. 4A and 4B, the processing such as the extraction and amplification of the nucleic acid is carried out in the sample chamber 16, and then, the solution is fed to the sample storage chamber 18 (namely, a storage mode). Thereafter, the solution containing the sample 2 in the sample storage chamber 18 is fed from the sample storage chamber 18 to the detecting part 20 as to be described below (namely, a sample supply mode).

The sample storage chamber 18 shown in FIG. 4B is configured such that, at the time of storage (in the storage mode), a plurality of samples 2 to be stored are integrated, and an output port 24 formed in the sample storage chamber 18 is arranged on the position higher than the liquid level when the sample is temporarily stored on the basis of a direction of gravity. Accordingly, the output port 24 is coupled not to a liquid in the sample storage chamber 18 but to a space in the sample storage chamber 18. The sample storage chamber 18 is arranged such that the output port 24 contacts the liquid in the sample storage chamber 18 when supplying the sample (namely, in the sample supply mode).

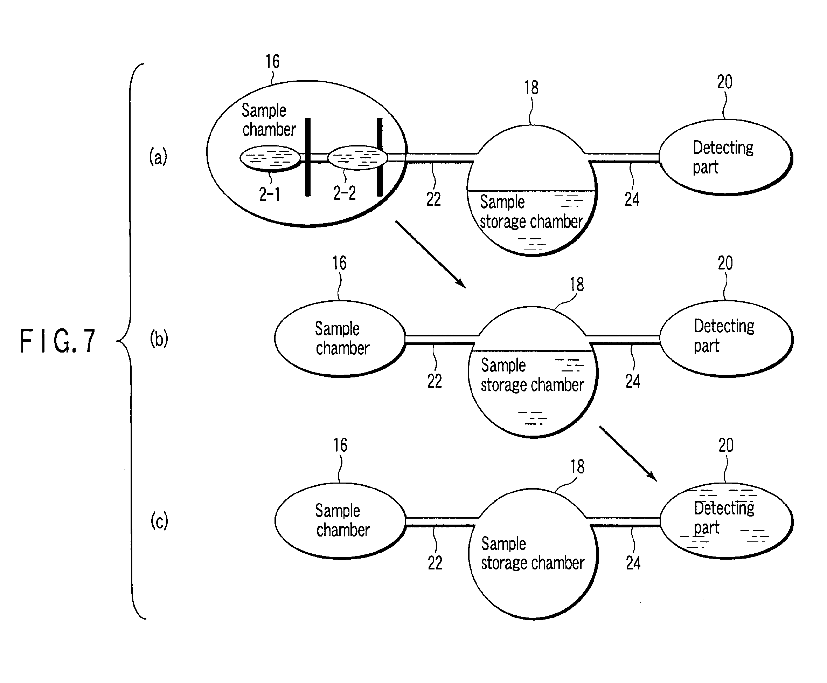

As shown in FIG. 7(a), the first and second samples 2-1 and 2-2 are held in the sample chamber 16, and due to the action of the pump part 8, the first and second samples 2-1 and 2-2 are sequentially supplied to the sample storage chamber 18 via an input port 22. As a result, if a reagent, namely, a buffer solution is held in advance in the sample storage chamber 18, the sample storage chamber 18, when storing the sample (namely, in the storage mode) is filled with a liquid obtained by mixing the first and second samples 2-1, 2-2, and the reagent as shown in FIG. 7(b).

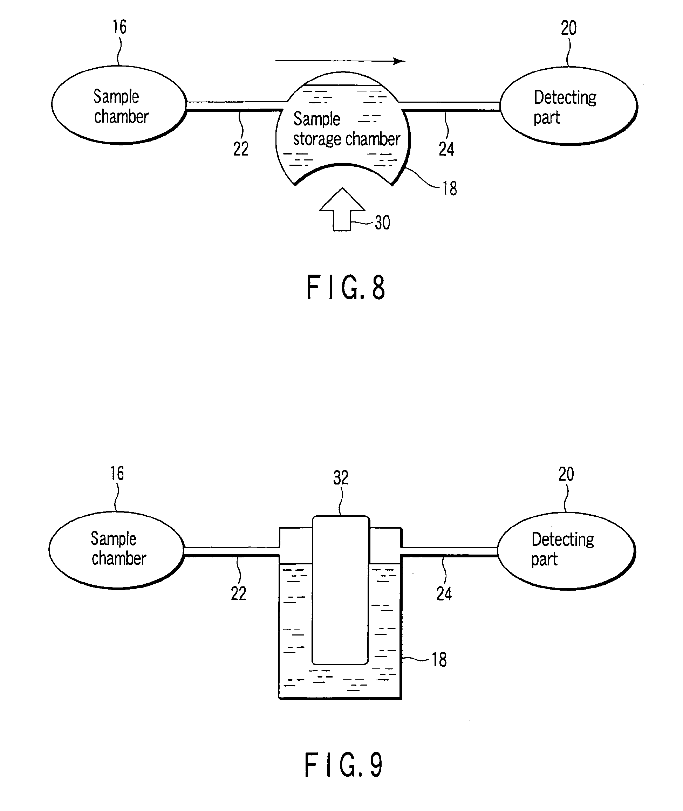

In the case of feeding a mixed liquid stored in the sample storage chamber 18 to the detecting part 20 (namely, in the sample supply mode), the sample storage chamber 18 is formed by a flexible container. In this case, as shown in FIG. 8, a pressure is applied from the outside as represented by an arrow 30, thereby deforming the sample storage chamber 18 so as to reduce its capacity. Then, the liquid level of the liquid in the sample storage chamber 18 is raised due to reduction of the capacity, and operating the pump part 8 in this state allows the liquid to be fed to the detecting part 20 as shown in FIG. 7(c).

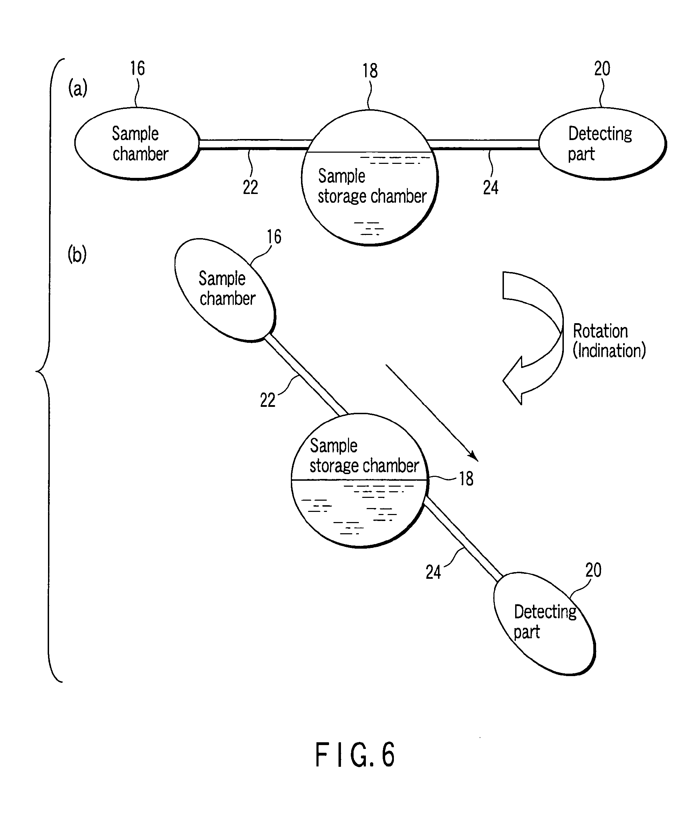

According to the example shown in FIG. 6(a), the nucleic acid detection cassette is kept substantially horizontally and all liquids to be integrated are integrated in the sample storage chamber 18 when storing the sample (in the storage mode). Thereafter, when supplying the sample (in the sample supply mode), the nucleic acid detection cassette is rotated by the nucleic acid detection system as shown in FIG. 6(b), and the posture thereof is inclined. By inclining the nucleic acid detection cassette, the output port 24 to be coupled to the detecting part 20 is positioned under the liquid level of the solution in the sample storage chamber 18 to be communicated to the solution. In this state, the cassette may be constituted in such a manner that, if the pump part 8 is operated, the solution is fed to the detecting part 20 by the pressure applied to gaseous body as represented by the arrow.

A method of deforming the sample storage chamber 18 to raise the liquid level may be configured as follows. In the case of deforming the sample storage chamber 18 from a sphere to a half moon to reduce its capacity as shown in FIG. 8, the sample storage chamber 18 is deformed by being pressed from the side face of the sample storage chamber 18 by means of a pressing section 32 as shown in FIG. 9, so that the capacity of the sample storage chamber 18 is reduced so as to raise the liquid level.

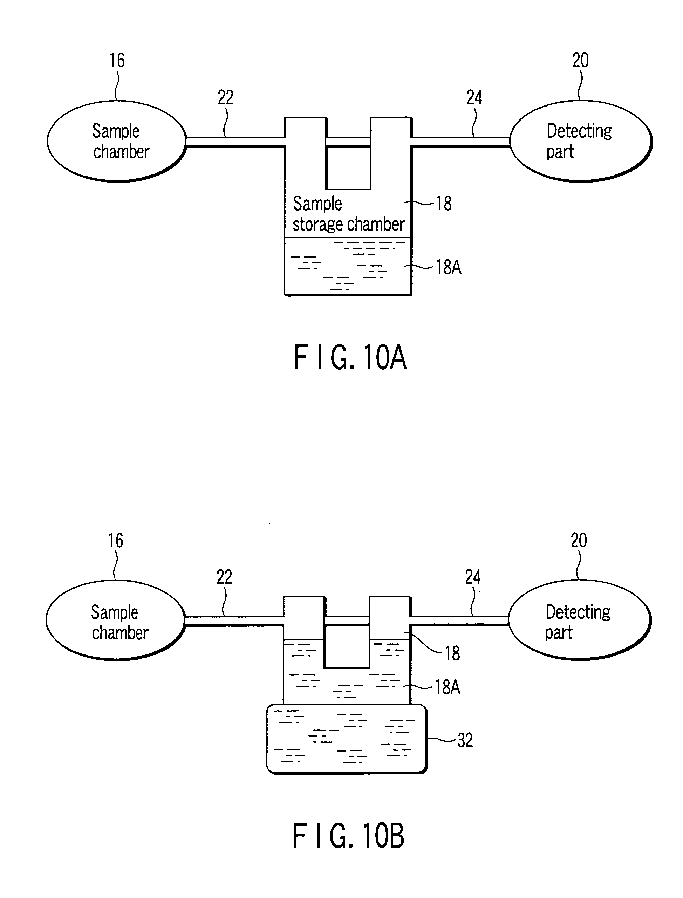

A modification is available that, as shown in FIG. 10A, the sample storage chamber 18 is formed into a box shape having a recessed center part, a lower part 18A of this box shape is made of a flexible material, and the lower part 18A is modified. In other words, when as shown in FIG. 10B, the lower part 18A of the sample storage chamber 18 is pushed up from the lower part by the pressing section 32, the liquid level in the sample storage chamber 18 is raised, and the sample storage chamber is filled with the liquid in the direction of the channel, so that the solution may be fed to the detecting part 20.

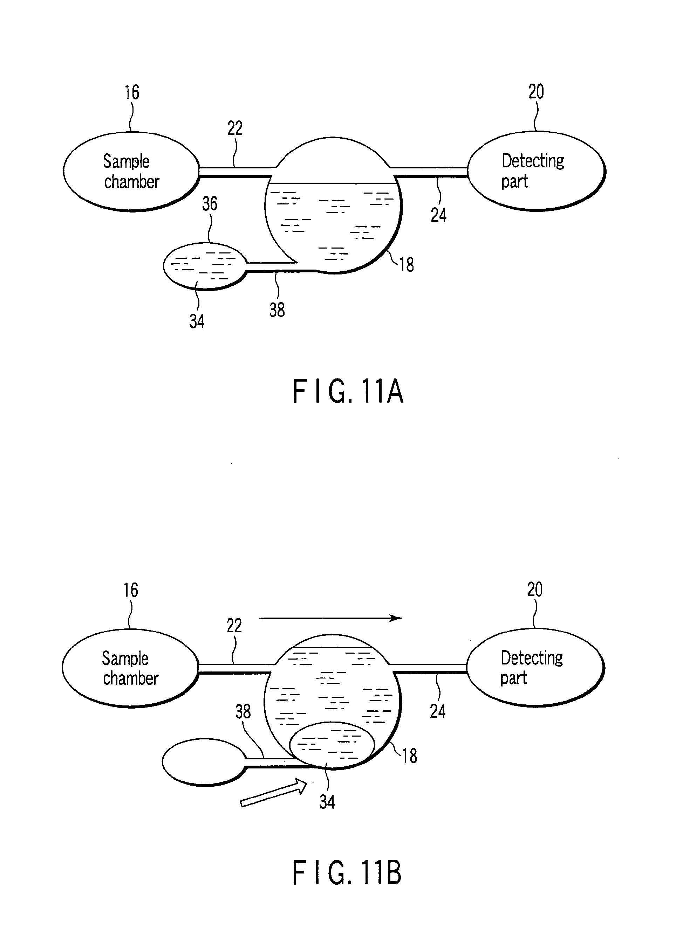

Further, as a modification, as shown in FIG. 11A, the sample storage chamber 18 may be constituted so as to have insolubility for the sample solution and an auxiliary liquid supply chamber 36 in which an auxiliary liquid 34 with a high specific gravity is housed is communicated to the sample storage chamber 18 via a communication path 38. In the cassette having the structure as shown in FIG. 11A, the solution containing the sample 2 is stored in the sample storage chamber 18 when storing the sample (in the storage mode), as same as the sample storage chamber 18 as shown in FIG. 4B. When supplying the sample (in the sample supply mode), the auxiliary liquid 34 is supplied from the auxiliary liquid supply chamber 36 to the sample storage chamber 18 via the communication path 38 as shown in FIG. 11B. In the sample storage chamber 18, the auxiliary liquid 34 has a higher specific gravity as compared to the sample solution and has insolubility for the sample solution. For this reason, the auxiliary liquid 34 is integrated in the lower part of the sample storage chamber 18, which results in raising the liquid level of the solution in the sample storage chamber 18. If the pump part 8 is operated in this state, the solution is fed to the detecting part 20 in the same way as the state shown in FIG. 7(c).

According to the above explanation, only one sample storage chamber 18 is arranged in the cassette. However, a plurality of sample storage chambers 18 may be arranged by being coupled with each other or branched. For example, when adopting a LAMP method as an amplification method, the following two kinds of reagents are prepared preferably.

```
(E) A boiled liquid (1 × reaction Mix + primer)


(F) An enzyme liquid (1 × reaction Mix + enzyme)
```

A LAMP amplification reaction may be practiced in such a manner that the two kinds of reagents are stored in different chambers and after injecting a sample such as a whole blood into a boiled liquid to be subjected to heat treatment, the boiled liquid is unified with the enzyme liquid.

Going through the steps as shown in the above-described examples, the following effects are obtained.

Upon heat treatment for cell destruction, a denaturation by heat of a primer can be made at the same time. It is difficult to feed the enzyme because the enzyme is present in minute amount and has a high viscosity. However, by mixing the enzyme with the boiled liquid by means of Reaction Mix, it is possible to increase the amount of the enzyme and to lower the viscosity of the enzyme, so that the enzyme can be smoothly fed. Since liquidities of the boiled liquid and the enzyme liquid are equal, both of them are easily mixed even if the complex mixing operation is not provided.

The sample storage chamber can be used also for integration of the boiled liquid and the enzyme liquid according to the above-described example. In this case, it is preferable that a temperature control mechanism is also added to the sample storage chamber 18 from the outside.

In addition, a sample storage chamber for carrying out another processing may be present among the sample storage chamber 18, the sample chamber 16, and the detecting part 20. Examples of the another processing include a RNA transcription reaction, a single-strand enzyme treatment, and a modification treatment by a fluorescence dye, an enzyme or the like.

The structure of the above-described nucleic acid detection cassette is simple so as to increase a reliability of inspection. A complex channel or valve is not required, which makes it possible to prevent an accident such that the sample is absorbed to the channel or valve and the sample amount is reduced or the sample density is lowered.

Further, since the liquid is stored in the chamber 18 by using gravity, the structure which is vertically long as a shape of a chamber can be realized. Accordingly, the nucleic acid detection cassette is prevented from extending in a planar direction structurally, so that it is possible to reduce a projection area when viewed from the above and make treatment of the cassette easy.

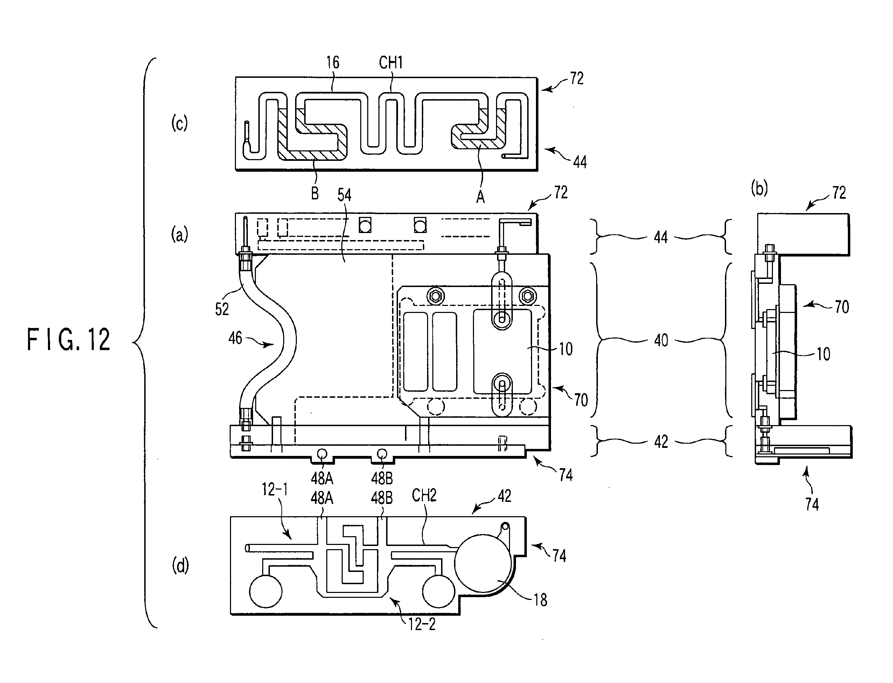

A nucleic acid detection cassette more concretized on the basis of the above embodiment and a nucleic acid detection system using the nucleic acid detection cassette will be described below with reference to FIGS. 12 to 19.

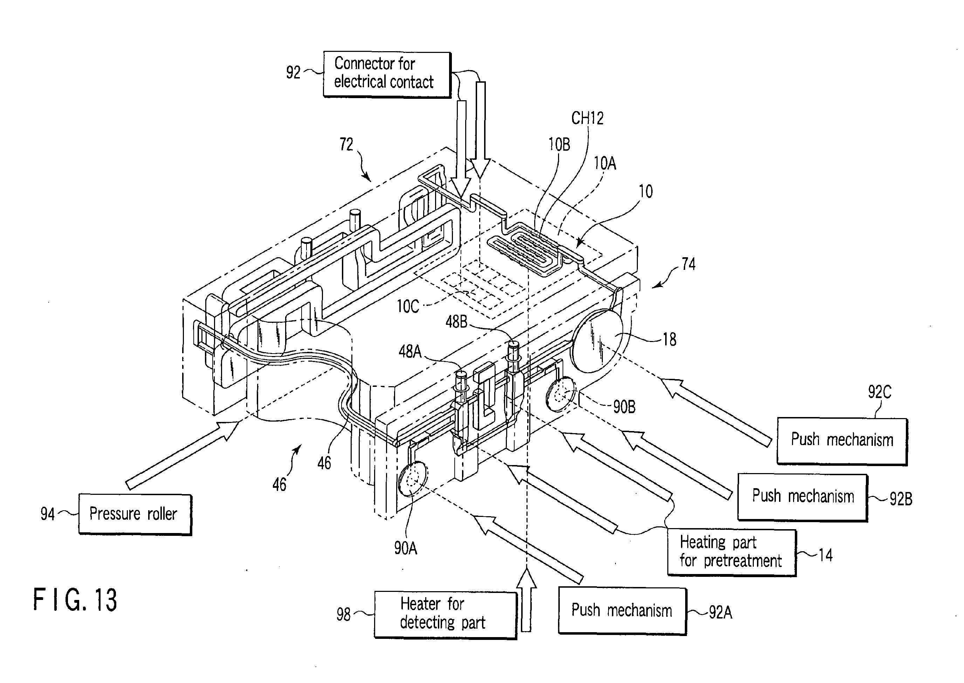

FIG. 12 is a exploded plan view showing the entire structure of a nucleic acid detection cassette according to a first embodiment of the present invention. Here, FIG. 12(a) is a plan view schematically showing the upper surface of the nucleic acid detection cassette of the first embodiment, and FIG. 12(b) is a front view of the nucleic acid detection cassette shown in FIG. 12(a). FIG. 12(c) is a plan view showing the inner structure of a reagent reservoir provided on one side of the nucleic acid detection cassette shown in FIG. 12(a), and FIG. 12(d) is a plan view showing the inner structure of a nucleic acid extraction and amplification section provided on the other side of the nucleic acid detection cassette shown in FIG. 12(c). FIG. 13 is a perspective view schematically showing the inner structure of the nucleic acid detection cassette shown in FIG. 12.

In FIG. 13, each part of the nucleic acid detection system for operating the nucleic acid detection cassette is shown by a block.

The nucleic acid detection cassette shown in FIG. 12 and FIG. 13 is equivalent to one specific example of the nucleic acid detection cassette shown in FIG. 5. The nucleic acid detection cassette is formed by an upper surface structure 70 and side face structures 72 and 74 provided on the opposite sides of the upper surface structure 70, and the entire shape thereof is made into substantially a saddle shape.

A detecting part 40 incorporating the DNA chip 10 and the channel CH 12 is arranged in the upper surface structure 70. Also, a pump part 46 is provided in the upper surface structure 70.

On one side face structure 74, provided are the sample chambers 12-1 and 12-2, the channel CH2, and the sample storage chamber 18.

The channel CH1 having the reagents A and B is provided on the other side face structure 72. In the side face structures 72 and 74 of the nucleic acid detection cassette, the sections of the channels CH1 and CH2 are determined to be the sizes which allow the channels CH1 and CH2 to be completely closed by the surface tensions of the sample and the reagent. Accordingly, the sample and the reagent can move through the channels CH1 and CH2 and the detecting part due to a pressure applied to gaseous body by the pumping action of the pump part 46.

<With respect to upper surface structure 70>

The DNA chip 10 is arranged on the upper surface structure 70. The DNA chip 10 has the structure such that a plurality of individual electrodes, a counter electrode facing the individual electrodes, and further, a reference electrode arranged in the DNA chip 10 and a probe DNA is provided on each individual electrode. On a substrate 10A of the DNA chip 10, formed is a probe channel CH12 as a detection chamber to be coupled to the channels CH1 and CH2. Electrodes including the individual electrodes 10B, the counter electrode and the reference electrode are arranged on the substrate 10A along the probe channel CH12, and a liquid is fed through the individual electrodes 10B and the gap between the counter electrode and the reference electrode. Wiring of the DNA chip 10 is connected to the individual electrode 10B, the counter electrode and the reference electrode, which are provided on the substrate 10A, and this wiring is also connected to electrode pads 10C. The electrode pads 10C are arrayed to be exposed on the upper surface structure 70 to be contact-connected to an electrode connector 92 on the side of the nucleic acid detection system upon detection of a current.

Preferably, a liquid detection sensor (not shown) for detecting liquid feed is arranged in the channels CH1, CH2, or CH12 in the vicinity of the DNA chip 10. In addition, preferably, the liquid detection sensor can discriminate the reagent A, the reagent B and the sample 2, can detect the liquid feed as well as determine the kind of the fed liquid.

As described in JP-A 2004-12577 (KOKAI), the channel CH12 of the detecting part 40 is formed by bonding, for example, a silicone rubber having a groove as a channel and the substrate of the DNA chip.

As shown in FIG. 12(a) and FIG. 13, in the upper surface structure 70 provided is the pump part 46 for feeding the liquid to the channel for coupling a nucleic acid extraction and amplification section 42, the detecting part 40, and a reagent reservoir 44.

The pump part 46 is provided on a base 54 having a surface curved so as to allow a pressure roller 94 to run thereon, and an elastic piping tube 52 which is a pump channel arranged so as to be exposed along the curved surface. The pressure roller 94 and the elastic piping tube 52 constitute the pump part 46 for feeding a liquid. When the pressure roller 94 runs on the elastic piping tube 52 and pressurizes the elastic piping tube 52 along the curved surface, the reagents A and B and the sample 2 are fed in the forward direction FW or in the backward direction BW. More specifically, the pressure roller 94 mounted on the side of the nucleic acid detection system main body is made in contact with the elastic piping tube 52, and the pressure roller is rotated to thereby modify the elastic piping tube 52, so that gaseous body or a liquid contained in the tube is supplied so as to be squeezed. As a result, the pressure roller is moved without directly contacting the liquid in the cassette channel as being understood from its structure. By reversing the rotational direction of the pressure roller 94, the liquid feeding direction is reversed. The liquid in the interior of the tube can be moved by operating the rotation of the pressure roller 94 from the outside. For this reason, without contact between the interior of the cassette and the outside in the pump part 46, it is prevented that the external environment is contaminated by the liquid in the interior of the cassette. The elastic piping tube 52 is communicated to the channels CH1 and CH2 of the side face structures 72 and 74.

<With respect to side face structure 74>

The side face structure 74 of the nucleic acid detection cassette has the nucleic acid extraction and amplification section 42 having two sample injection ports 48A and 48B as shown in FIG. 12(d) and FIG. 13.

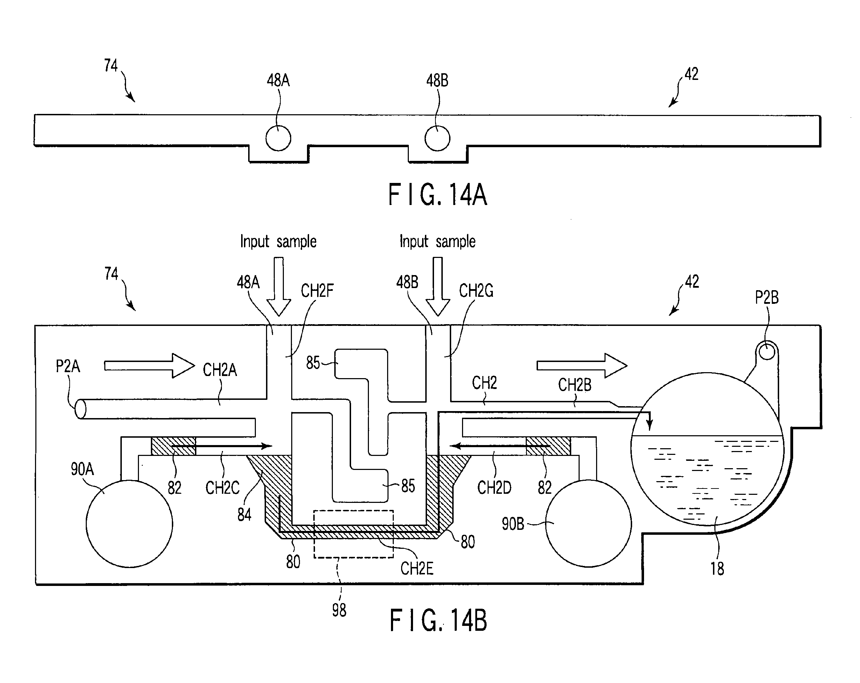

The nucleic acid extraction and amplification section 42 shown in FIG. 12(d) has the structure shown in FIGS. 14A and 14B as to be described in detail, and has the channel CH2 defined in its interior. The nucleic acid extraction and amplification section 42 is required to modify a member for controlling the movement of the liquid, and it is formed by an elastic body such as a silicone rubber.

Here, a constructional material is not always limited to a silicone rubber, and examples thereof include a fluorine rubber represented by FKM or FPM and an ethylene-propylene rubber represented by EP, EPDM, and EPT. In addition, the constructional material is not limited to a rubber, but it may have a structure such that a part thereof can be modified not forming a rigid box shape, by use of a thin PET film, a PP film, a polyvinylchloride film, or a PE film.

FIGS. 14A and 14B show a detailed structure of the nucleic acid extraction and amplification section 42.

An input port P2A of the side face structure 74 is opened to a channel CH2A as well as is connected to the elastic piping tube 52, and an output port P2B of the side face structure 74 is opened to a channel CH2B as well as is connected to the channel CH12 on the DNA chip 10.

As an example, the nucleic acid extraction and amplification section 42 is entirely formed by a silicone rubber. As shown in FIG. 14A, two sample injection ports 48A and 48B are formed so that their openings are opened to the upper face of the nucleic acid extraction and amplification section 42.

Examples of the sample 2 include a blood, a hair root, a nail, a fingerprint, an oral mucous membrane, and a cell in the case of a sample of a human being and a sample of an animal origin. Other examples thereof include a virus, a mold, and a plant cell. Further, it is also possible to use ones obtained by applying nucleic acid extraction processing such as boiling to these samples in advance. The nucleic acid extraction and amplification section 42 is preferably kept in a seal state, after injecting the sample 2 into the nucleic acid extraction and amplification section 42.

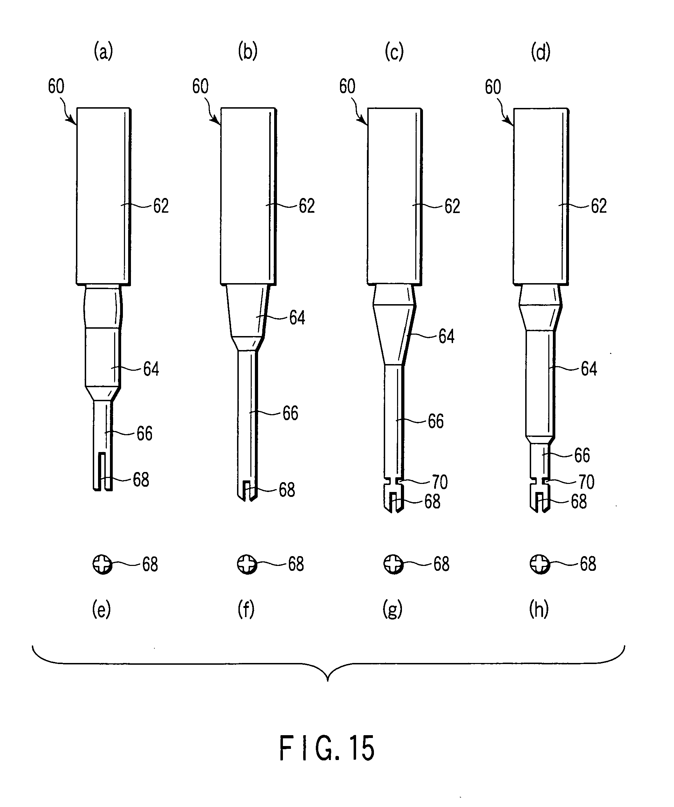

Upon treatment of the sample 2 which is a liquid as represented by a blood, a sampling bar 60 for sampling the sample 2 as shown in FIG. 15. The sampling bar 60 has a columnar base 62 for grasping the sampling bar 60, and a middle body 64 is elongated from the base 62. As shown in FIG. 15, the middle body 64 may have various shapes having different entire lengths, and the middle body 64 is formed in a substantially tapered shape. According to need, a part of the columnar middle body 64 is inserted in the sample injection ports 48A and 48B. In the same way, a needle-like part 66 is elongated from the middle body 64. In the case where the sample injection ports 48A and 48B are closed by a film in airtight, the needle-like part 66 has a small diameter to such an extent that it can stick out of the film to enter the interiors of the sample injection ports 48A and 48B. As shown in FIG. 15, a slit 68 is formed in a cross shape on the front end of the needle-like part 66, and the sample 2 can be attached to the slit 68. FIG. 15 shows the shapes of the front-end surfaces of FIG. 15, respectively, and the surfaces are formed in substantially the same shape. As shown in FIG. 15, the needle-like part 66 is provided with different entire lengths in accordance with samples to be sampled respectively, and the length of the slit 68 is different depending on the sample to be sampled. As shown in FIG. 15, only the slit 68 may be formed in the needle-like part 66. Alternatively, as shown in FIG. 15, the needle-like part 66 may be have, in addition to the slit 68, a ring-like recessed part 70 arranged therearound so that the sample 2 is sampled by the slit 68 and the ring-like recessed part 70. In addition, the front end of the sampling bar 60 may be formed only by the ring-like recessed part without the needle-like part. A plurality of such ring-like recessed parts may be formed.

The sample 2 is attached to the front end of the sampling bar 60 to be inserted into the sample inputting ports 48A and 48B, and then, the sample 2 is injected in the channel CH2. The dimensions or the numbers of the slits 68 and the ring-like recessed parts 70 are determined so that the slit 68 formed on the front end of the sampling bar 68 or the slit 68 and the ring-like recessed part 70 can hold the sample 2 of an appropriate quantity (for example, about 1 μL).

As shown in FIG. 14B, a reagent 80 for pretreatment such as one for extracting a nucleic acid is charged in advance in a sample chamber 84 defined in the channel CH2 in the sample injection ports 48A and 48B, and the front end of the needle-like part 66 of the sample-sampling bar 60 is soaked in the reagent 80. In addition, a reagent holding area is defined in advance in the channel which is the channel CH2 and branched from the sample chamber 84. The reagent holding area stores a reagent 82 for pretreatment which is a different kind from the reagent 80, such as for example, a reagent for amplification, so as to close the channel CH2. After the nucleic acid extraction processing is carried out for the sample, for example, by the reagent 80 contained in the sample chamber 84, the reagent 82 in the reagent holding area of the branched channel is added to the sample so as to be provided for the amplification processing. It is preferable that the reagent 82 for amplification is housed in another area in this way because the reagent 82 has a low heat resistance in some cases.

Before being provided for measurement, the reagents 80 and 82 are also frozen in the cooled cassette as the reagents A and B, and the cassette with the frozen reagents 80 and 82 is provided for delivery or the like. Accordingly, the reagent 82 and the reagent 80 are retained being frozen in the reagent holding area and the sample chamber 84, respectively, so that the reagents 82 and 80 are prevented from being mixed in another reagent due to a disturbance or the like to be given to the cassette upon delivery. Upon measurement, the frozen reagents 80 and 82 are returned to a normal temperature to be made into a liquid and then attached to the detection system. In the detection system, the side face structure 74 is kept in a state of standing right in substantially a vertical direction along approximately the direction of gravity, so that the reagent 80 is to be retained in the sample chamber 84 by the effect of the gravity.

As described above, the sampling bar 60 shown in FIG. 15 is inserted into the injection ports 48A and 48B, so that the sample injection ports 48A and 48B are sealed from the outside. When in this state, the heating sections 14-1 and 14-2 heats the sample 2 mixed in the reagent 80, for example, for five minutes or more at 95°C, the nucleic acid in the sample 2 is extracted. With respect to the reagent (enzyme) 82 having no heat resistance, for example, the LAMP amplification enzyme, the reagent (enzyme) 82 is stored in the reagent holding area of the channel CH2 which is separated from the heating area as shown in FIG. 5. After the above-described heat treatment is terminated, the reagent 82 is added to the heated sample 2. The amount of the reagent 82 to be added is minute, for example, about 5 to 20 μL. For this reason, providing a local liquid feeding mechanism as shown in FIG. 5 may realize feeding of the reagent 82 without using a liquid feeding pump of the entire cassette. In the structure shown in FIG. 14B, press pumping mechanisms 90A and 90B which are communicated to the channel CH2 are arranged in the nucleic acid extraction and amplification section 42, and these press pumping mechanisms 90A and 90B realize feeding of the reagent 82. The press pumping mechanisms 90A and 90B are formed in a cavity structure having a recessed interior of the pump part. By pressing a circle part closing this cavity structure from the outside by means of push mechanisms 94A and 94B shown in FIG. 13, the inner capacity of the pump part is compressed. As a result, the reagent 82 contained in the reagent holding area of the channel CH2 is pushed out to channels CH2F and CF2G, respectively. Gaseous body, for example, air is moved to pressure buffers 85 for press pump which are communicated to the channels CH2F and CH2G, respectively, and the reagent 82 flows into a heated sample (+ reagent 80) in the channels CH2F and CH2G. Thereafter, the heat treatment for amplification is carried out. Upon the LAMP amplification using the LAMP amplification enzyme as the reagent 82 described here, the nucleic acid can be typically amplified by holding the LAMP amplification enzyme for about one hour at a temperature in the range of 55 to 65°C. It is not always necessary to provide the pressure buffer 85 for press pump.

Here, assume that the present cassette is stored for a long period of time with the reagent 82 held by the cassette. In this case, freezing is preferable as described above in order to make it harder for the reagent to be deteriorated. However, since gaseous body is 14 argely expanded and contracted due to lowering of a temperature in accordance with a gaseous body state equation, PV = nRT, this should be considered. For example, in the present cassette, freezing the sample injection port 48 as being opened to the outside makes it possible to reduce the influence of contraction of air in the channel and the chamber which are connected to the sample injection port 48. However, since air in the press pumping mechanisms 90A and 90B is not connected to the outside, the reagents 82 are moved due to contraction of its volume, and it can be considered that the reagents 82 may fall in the press pumping mechanisms 90A and 90B. Accordingly, it is preferable that the volumes of the press pumping mechanisms 90A and 90B are not so large. However, the volume enough to push out the reagent 82 is required. It is preferable to satisfy the following formula:

$$[(T1 - T2)/(273 + T1)] \times (A + B) \leq B$$

$$(C + D) \leq A$$

where A denotes a volume of the press pumping mechanism 90; B denotes a channel volume between the press pumping mechanisms 90A and 90B and the reagent 82; C denotes a volume of the reagent 82; D denotes a channel volume between the reagent 82 and the chamber having the reagent 80 stored therein; T1 denotes a temperature when charging the reagent; and T2 denotes a temperature when freezing the reagent.

Assuming that, in the above-described two formulas, the temperature T1 when charging the reagent is in the order of a room temperature 25°C and the temperature T2 when freezing the reagent is in the order of a refrigerator temperature -20°, the following formula is satisfied.

$$(C + D) \leq A \leq 5 \times B$$

<With respect to side face structure 72>

The side face structure 72 of the nucleic acid detection cassette has a structure that the reagent reservoirs 44 (44A, 44B) are arranged on the opposite sides thereof as shown in FIG. 12(c).

As shown in FIG. 12(c), in the reagent reservoir 44, a buffer solution as the reagent A and an intercalator as the reagent B are stored in the channel CH1 via the gaseous body 16 in this order from an inflow port side to the detecting part 40 (upstream side). For convenience of housing plural kinds of reagents, the channel CH1 desirably snakes. In other words, it is preferable that, when mounting the nucleic acid detection cassette on a horizontal plane, an area where the direction of the channel is substantially horizontal to the gravity direction and an area where the direction of the channel is substantially vertical to the gravity direction are provided and both the reagents A and B are arranged in the former area. This enables to stably hold the liquid. In this case, for stably holding the liquid in each reagent reservoir, it is preferable that the channel section of the former area is smaller than the channel section of the latter area. Before being provided for measurement, the reagents A and B are frozen in the cooled cassette, and the cassette with the frozen reagents A and B is provided for delivery or the like. Accordingly, the problem that the reagent A and the reagent B are mixed due to disturbance or the like given to the cassette upon delivery is prevented. In addition, the reagents A and B frozen upon measurement are returned to a normal temperature to be made into a liquid and then attached to the detection system. When in the detection system, the side face structure 72 is kept in a state of standing right in substantially a vertical direction, the reagent A and the reagent B are retained in the reagent reservoirs 44A and 44B by the effect of the gravity.

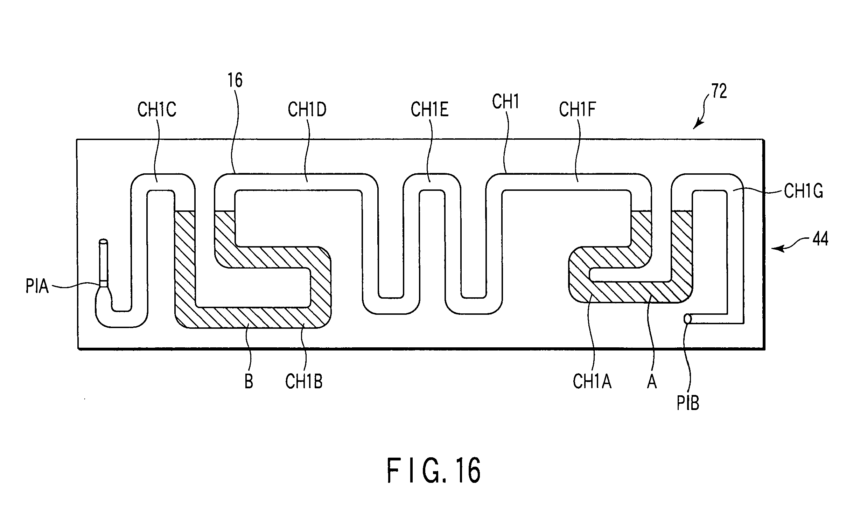

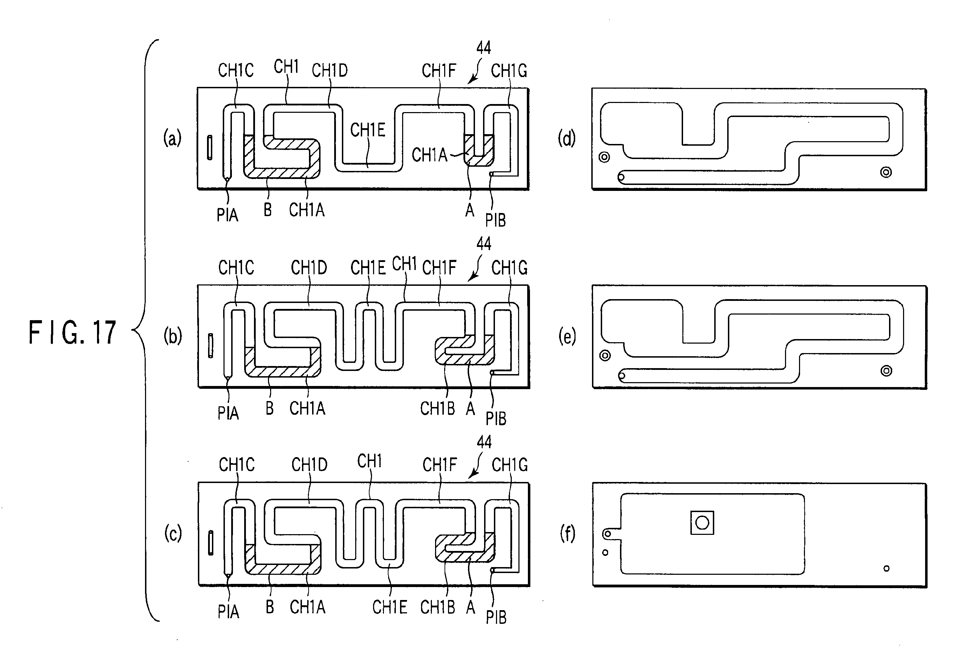

The reagent reservoir 44 shown in FIG. 12(c) has the structure shown in FIG. 16 or shown in FIG. 17 as a modification in detail, and the channel CH1 is defined in the reagent reservoir 44.

The member of the reagent reservoir 44 is preferably deformed in response to expansion or contraction of the reagent A and the reagent B. The reagent reservoir 44 has the structure that the channel CH or the like is formed on a hard chip substrate as a groove, the surface of the substrate is covered with an elastic body such as a silicone rubber, a substrate as shown in FIG. 17 is arranged on the rear surface of the chip substrate, and a substrate on which a buffer space for absorbing expansion or contraction of the reagent A and the reagent B is arranged, is pasted to the chip substrate.

Here, a coating material is not always limited to a silicone rubber but the coating material may be a fluorine rubber represented by FKM or FPM and an ethylene-propylene rubber represented by EP, EPDM, and EPT. In addition, the coating material is not limited to a rubber, but it may have a structure such that a part thereof can be modified not forming a rigid box shape, by use of a thin PET film, a PP film, a polyvinylchloride film, or a PE film.

In the side face structure 72 of the nucleic acid detection cassette as shown in FIGS. 12 and 16, an input port P1A is opened to the channel CH1 as well as is connected to the elastic piping tube 52, and an output port P1B of the side face structure 72 is opened to the channel CH1 as well as is connected to the probe channel CH12 (detection chamber) on the DNA chip 10. The channels CH1A and CH1B having the reagent A and the reagent B stored therein have larger channel widths, namely, larger channel sections as compared to other channels CHIC to CH1G.

Further, not limited to the form of the channel CH1 as shown in FIGS. 12(c) and 16, the reagent reservoir 44 has various forms to define the channel CH1. Particularly, it is preferable that the reagent reservoir 44 has a buffer cavity part, which is communicated to the channel CH1, for absorbing a pressure expansion in the cassette. Providing the buffer space provides the following advantage. For example, when feeding the sample solution from the sample storage chamber 18 to the detecting part 40, the sample storage chamber 18 may be deformed by a push mechanism 92C to decrease the inner volume of the cassette. Even in this case, the buffer space provided absorbs a pressure. Therefore, it is possible to prevent leakage to the outside of the cassette, and the reagent can move in the channel CH1 smoothly.

A plate unit forming the side face structure 72 can be configured by a first substrate plate (for example, FIG. 17) on which a groove or a hole to be made into the channel CH1 is formed; a second substrate plate (not shown) which is pasted on one surface of the first substrate to define the channel CH2; and a third substrate plate (for example, FIG. 17) which is pasted on the other surface of the first substrate plate to define the channel CH1. FIG. 17 (d) to (f) correspond to FIG. 17 (a) to (c), respectively, and they show a plate unit which is arranged on the rear surface of the plate unit for defining the channel CH1 shown in FIG. 17 (a) to (c) and is capable of defining the buffer space communicated to the channel. It is preferable that a part or entirety of the second plate unit is formed by an elastic member, and the volume of the sample storage chamber is variable due to the effect from the outside.

In addition, it is preferable that the second plate unit includes a fourth reagent reservoir, which is communicated to the sample chamber and stores a fourth reagent to be supplied to the sample chamber, and that the fourth reagent reservoir is formed by an elastic member and has its volume variable due to the effect from the outside.

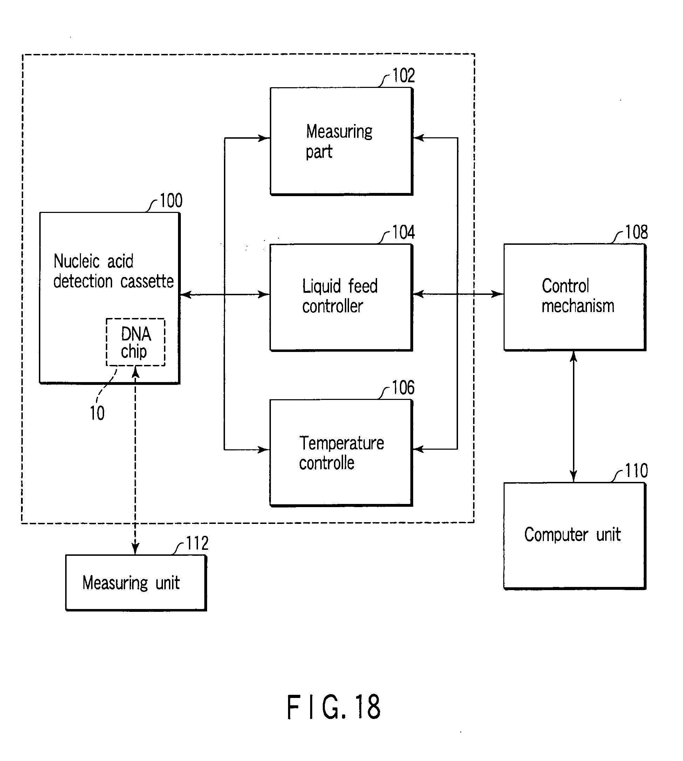

Next, a nucleic acid detection system for carrying out detection of a nucleic acid by inserting and assembling the nucleic acid detection cassette therein will be described with reference to FIG. 18.

As shown in FIG. 18, the nucleic acid detection system includes a measuring section 102 for measuring temperatures or the like of the reagents A and B and the sample 2 contained in a nucleic acid detection cassette 100, and a liquid feed controller 104 for controlling liquid feed of the reagents A and B and the sample 2 contained in the channels CH1 and CH2. The liquid feed controller 104 includes the pressure roller 94 described above, and push mechanisms 92A, 92B and 92C. Further, the nucleic acid detection system is provided with a temperature controller 106 for controlling the temperature of the sample 2 by controlling the temperatures of the heating sections 14-1 and 14-2. The temperature controller 106 includes a detecting part heater 98 for heating the DNA chip 10 to control the temperature of the sample or the reagent flowing thorough the channel CH12 in the DAN chip 10. These measuring section 102, liquid feed controller 104, and temperature controller 106 are controlled by a control mechanism 108 under the control of a computer unit 110. The nucleic acid detection system is further provided with a measuring unit 112 for measuring a reaction generated in the DNA chip 10. The measuring unit 112 detects a detection signal from the electrical contact pad 10C by using the electrical contact connector 92 shown in FIG. 13 and specifies an electrode 10B to be conducted, thereby specifying the sequence of the sample nucleic acid.

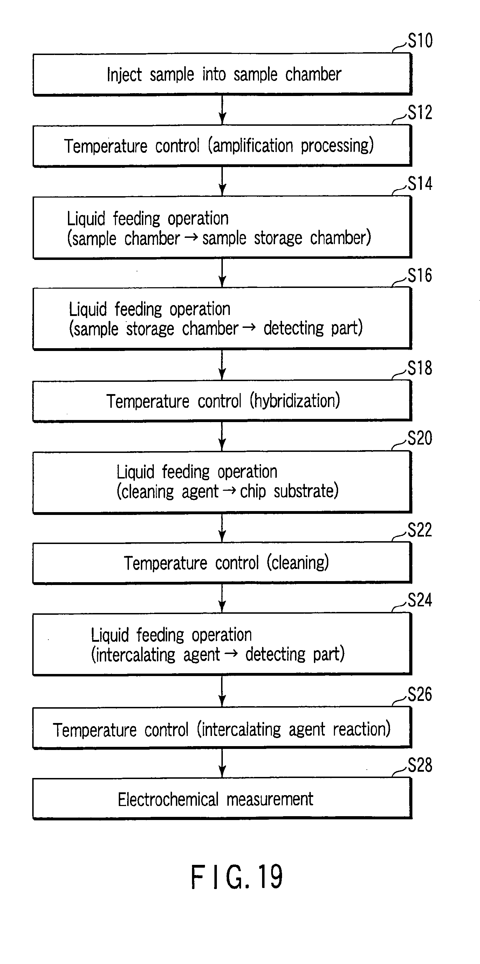

The detection will be executed in the procedure, for example, as shown in FIG. 19 by using such a nuclear acid detection system and nucleic acid detection cassette (refer to FIGS. 12, 13, 14A, 14B, 15 and 16).

First, the sample-sampling bar 60 is inserted in the sample injection ports 48A and 48B, and the sample is mixed in the reagent 80 of the sample chamber 84 (step S10).

Next, the sample is input in the reagent 80, and the extraction and amplification processing is carried out. In this case, the temperature is controlled so as to be an appropriate temperature by the heating section 14 (step S12).

Thereafter, the pressure roller 94 is operated to feed a sample solution containing the amplified sample to the sample storage chamber 18 via the channel CH 2 (step S14).

In the sample storage chamber 18, the reagent stored in advance therein and the sample solution containing the sample 2 are mixed with each other. Here, the output port P2B is closed by the mixed solution with the nucleic acid detection cassette 100 being inclined, and at the same time, the push mechanism 92 is operated. As a result, the mixed solution is supplied from the sample storage chamber 18 to the channel CH12 on the DNA chip 10 (step S16).

In this DNA chip 10, hybridization is generated with the temperature controlled (step S18).

Next, the pressure roller 94 is operated in the direction opposite to that in step S14, and the sample solution on the DNA chip 10 is returned from the DNA chip 10 to the sample storage chamber 18, so that the reagent A (washing reagent) is fed to the channel CH2 in the DNA chip 10 (step S20).

With the DNA chip 10 kept at a predetermined temperature, the interior of the DNA chip 10 is cleaned (step S22).

Further, the pressure roller 94 is operated in the same direction as that in step S22, and the reagent B (intercalator) is fed to the channel CH12 on the DNA chip 10 (step S24).

In the same way, a reaction by the intercalator is generated on the DNA chip 10 with the DNA chip 10 kept at a predetermined temperature (step S26).

With the intercalator reacted, an electric chemical reaction is measured to specify the nucleic acid of the sample (step S28).

These steps, the cassette, and the system will be described with supplement below.

According to the cassette structure including a plurality of sample injection ports 48A and 48B, it is preferable that the channel part CH2E communicating each injection port is clumped by the clip 98 as shown by a broken line so as to divide the sample chamber 84 into two. That is because this prevents the reagents 82 and 80 from suddenly moving in the cassette when transporting the cassette and further, prevents the reagents 82 and 80 from suddenly moving in the cassette in the same way when the balance of the inner pressure is disrupted upon injection of the cassette.

Upon heating of the sample 2, the heating devices 14, 14-1, and 14-2 arranged to the nucleic acid detection system from the outside contact the cassette. In this case, in order to limit the heating area, it is preferable that the channel CH2E is closed by the heating device arranged in the nucleic acid detection system for the communicated peripheral CH2A, CH2C, CH2B, and CH2D.

Let us consider the cassette structure provided with a plurality of sample injection ports 48A and 48B. In a step of adding the reagent 82 to the heated sample 80 by the press pumps 90A and 90B, closures of only the parts of the channel CH2C and CH2D where the corresponding samples are held are released, and then, the press pumps 90A and 90B are pressed to add the reagent 82 to the heated sample 80, whereby it is possible to prevent the liquid from involuntarily moving. The reagent 82 can be added to another sample according to the procedure that the above-described released channels CH2C and CH2D are closed again and the channels CH2C and CH2D for the corresponding samples are released to add the reagent.

When the sample solution containing the sample is fed to the sample storage chamber 18, the sample solution is spaciously divided by gaseous body. However, only a liquid remains in the sample storage chamber 18 and the liquid passes through the separated gaseous body. In this way, plural sample solutions are stored in the sample storage chamber 18 as an integrated liquid without being divided by gaseous body.

In FIG. 14B, the channel (CH2E) communicating the sample injection port 48A to the sample injection port 48B is illustrated so as to be located on the bottom parts of the sample injection ports 48A and 48B. However, arranging the CH2E on the higher position from the bottom part makes it possible to prevent the residual from flowing out to the channel by saving a residual generated when boiling a blood on the bottom of the injection port. Such a configuration enables to remove "blocking" of the channel due to the residual and an inhibitor in detection as much as possible.

In order to move the sample from the sample storage chamber 18 to the detecting part 40, there is a method of compressing the sample storage chamber 18 by means of a compression mechanism for compressing the sample storage chamber 18, the compression mechanism being mounted in the nucleic acid detection system. Thereby, the inner volume of the sample storage chamber 18 is decreased and the liquid level is raised. Consequently, it is possible to raise the liquid level upward from the channel CH2 communicated to the detecting part 40.

If stirring of various samples and reagents is needed other than integration of the various samples and reagents, they can be stirred by repeating compression and release of the sample storage chamber 18. By feeding the sample with the sample storage chamber 18 compressed, the sample in the chamber 18 is moved to the detecting part 40. Liquid feed of the sample is carried out by the pump 8 provided in a circulation channel.

With respect to liquid feed of the sample from the sample storage chamber 18, there are some methods other than the above-described method as already described. As described with reference to FIG. 8, there is a method of modifying the inner volume by pressing the lower part of the sample storage chamber 18 from the outside and raising the liquid level or there is a method of coating an outflow port of the channel CH2 with a liquid by inclining the channel CH2 in a gravity direction so as to be "downward" as described above with reference to FIG. 6 (a) and (b). In addition, there is a method of raising the sample liquid level by injecting a liquid 36 which is insoluble in the sample and has a high specific gravity from the channel 38 arranged on the lower part of the sample storage chamber 18 as shown in FIG. 11, or there is a method of raising the sample liquid level by modifying the sample storage chamber 18 from the lower part as shown in FIG. 10. Any method can be applied to feed of the liquid as long as the liquid surface can be raised in this way.

In the reagent reservoir 44, two kinds of reagents, namely, the reagent A and the reagent B are stored. In this case, if the direction of the channel in the reagent reservoir 44 is arranged horizontally to the gravity direction, the reagents A and B are moved in the interior of the reagent reservoir 44 when the cassette is only slightly inclined. Therefore, as shown in FIG. 16, a part of the channel in the reagent reservoir 44 is arranged so as to be vertical to the gravity direction and the housing areas of respective reagents A and B are configured so as to be a U-shaped part in the channel. This enables to stably hold the reagents A and B. However, it is necessary to feed the reagents A and B in a direction against the gravity in the channel structured on the vertical plane. In this case, if the sectional shape of the channel is not appropriate, the reagents A and B are not normally fed. The appropriate channel sectional shape is preferably less than 4 mm × 3mm, and more preferably less than 2 mm × 2mm depending on a viscosity of the reagent and a water-shedding quality of the cassette member, etc. The angular part of the section is preferably a round shape and a circle section is ideal. In addition, by making the channel section vertical to the gravity direction in the channels on the vertical planed smaller and making the channel section horizontal to the gravity direction having no influence of the gravity larger, it is possible to store large amounts of the reagents while keeping the normal liquid feed of the reagents. In this way, determining the section (the width) of the channel provides the advantage that, even if air bubbles are generated from the reagents A and B when the frozen reagents A and B are unfrozen, it is possible to smoothly remove the air bubbles among the reagents A and B.

(Example 1)

An example of use of the full automatic nucleic acid detection cassette according to the first embodiment will be specifically described below.

1. Preparation of nucleic acid detection cassette

The following reagents were prepared in the sample chambers 12-1 and 12-2 and the sample storage chamber of the nucleic acid detection cassette. Sample chamber 12-1: Enzyme for LAMP amplification reaction, Buffer for LAMP amplification reaction, Primer set A, dNTP
Sample chamber 12-2: Enzyme for LAMP amplification reaction, Buffer for LAMP amplification reaction, Primer set B, dNTP
Sample storage chamber 18: 2 × SSC The following reagents obtained by immobilizing a DNA probe were prepared for the nucleic acid immobilized chip.
Electrode A-1: ATGCTTTCCGTGGCA
Electrode A-2: ATGCTTTGCGTGGCA
Electrode B-1: ATGCTTTCCGTGGCA
Electrode B-2: ATGCTTTGCGTGGCA

2. The nucleic acid is detected full automatically. It was assumed that the following temperature control, liquid feed control, and detection were entirely programmed in the nucleic acid detection system.

Blood is collected from a human being, and 1 μL of whole blood is sampled by a pipette or the above-described sample-sampling bar to be injected in the sample chambers 12-1 and 12-2.

A temperature is controlled from the outside to perform the LAMP amplification reactions A and B.

At first, the chambers 12-1 and 12-2 are separated. After the LAMP reaction, the chambers are communicated with each other, and the LAMP productions A and B are moved to the sample storage chamber 18 by the liquid transmission mechanism to be made into a solution integrated with 2 × SSC stored in the sample storage chamber 18 in advance. In this case, the liquid level of the integrated solution is positioned to be lower than the input port 22 and the output port 24.

The sample storage chamber 18 is compressed by the compression mechanism, which is incorporated in the nucleic acid detection system, for compressing the sample storage chamber 18. Then, the liquid level of the integrated solution is raised to the higher position than the output port 24.

The compression of the sample storage chamber 18 is released, and the sample storage chamber 18 is compressed again. By repeating this compression and release, plural integrated solutions are mixed to be fed to the detecting part 20 (40) as a sample for detection (DNA sample).

The sample for detection is introduced in the detecting part 20 (40) to thereby start detection.

As a result, it has been found that the DNA in the sampled sample has the sequence of CTGCCACGGAAAGCAT.

(Example 2)

An example of use of the full automatic nucleic acid detection cassette according to the first embodiment will be specifically described below.

1. Preparation of nucleic acid detection cassette

The following reagents were prepared in the sample chambers 12-1 and 12-2 and the sample storage chamber 18 of the nucleic acid detection cassette.

Sample chamber 12-1: AmpDirect (manufactured by Shimadzu Corporation), Enzyme for PCR, Primer set A, dNTP

Sample Chamber 12-2: AmpDirect (manufactured by Shimadzu Corporation), Enzyme for PCR, Primer set B, dNTP

Sample storage chamber 18: 2 × SSC

The following reagents obtained by immobilizing a DNA probe were prepared for the nucleic acid immobilized chip.

Electrode A-1: ATGCTTTCCGTGGCA

Electrode A-2: ATGCTTTGCGTGGCA

Electrode B-1: ATGCTTTCCGTGGCA

Electrode B-2: ATGCTTTGCGTGGCA

2. The nucleic acid is detected full automatically. The following temperature control, liquid feed control, and detection are entirely programmed in the nucleic acid detection system.

Blood is collected from a human being and 1 μL of whole blood is sampled by a pipette or the above-described sample-sampling bar to be injected in the sample chambers 12-1 and 12-2.

A temperature is controlled from the outside to perform the PCR amplification reactions A and B.

At first, the chambers 12-1 and 12-2 are separated. After the PCR reaction, the chambers are communicated with each other, and the PCR products A and B are moved to the sample storage chamber 18 by the liquid feeding mechanism to be made into a solution integrated with T7 exonuclease stored in the sample storage chamber 18 in advance. Then, the solution is held for about 30 minutes, whereby a single-strand treatment of two strands of DNA is carried out. In this case, the liquid level of the integrated solutions is located at a lower position than the input port 22 and the output port 24.

The sample storage chamber 18 is compressed by the push mechanism 92C, which is incorporated in the nucleic acid detection system, for compressing the sample storage chamber. Then, the liquid level of the integrated solution is raised to the higher position than the output port 24.

The compression of the sample storage chamber 18 is released, and the sample storage chamber 18 is compressed again. By repeating this, the integrated plural solutions are mixed so as to be a sample for detection.

By introducing the sample for detection in the detecting part 20 (40), the detection of the nucleic acid is carried out. As a result, the DNA in the collected sample has the sequence of CTGCCACGGAAAGCAT.

As described above, the present invention is effective for a technical field of a nucleic acid detection cassette for full automatically carrying out steps for extraction, amplification, detection and the like of the nucleic acid in order to detect a target nucleic acid, and a nuclear acid detection system using the nucleic acid detection cassette.

As described above, according to the present invention, it is possible to provide a nucleic acid detection cassette having a structure for preventing contamination and having a convenient structure not requiring a valve or a complex channel, and a nucleic acid detection system.

**Claims**

1. A nucleic acid detection cassette, comprising:

   a first injection port which allows a sample containing a target nucleic acid to be injected therethrough;
   a first channel;
   a second channel which is communicated to the first channel and the first injection port;
   a detecting part comprising a detection chamber which is communicated with the first and second channels and is so formed as to be separated from the outside of the nucleic acid detection cassette, and a nucleic acid detection unit which detects an electric signal to identify the target nucleic acid in the sample supplied to the detection chamber, the detecting part being arranged between the first and second channels;
   a first reagent reservoir which is defined in the first channel and stores a first reagent while separating the first reagent from the sample via a gaseous body;
   a second reagent reservoir which is defined in the first channel and stores a second reagent while separating the second reagent from the first reagent and the sample via a gaseous body;
   a sample chamber which is defined in the second channel and is communicated to the first injection port, the sample chamber receiving the injected sample; and
   a pump part which is arranged between the first and second channels so as to be communicated to the first and second channels, the pump part forming a closed path together with the first channel, the second channel, and the detecting part, a pressure being applied to the pump part from the outside of the nucleic acid detection cassette so that the sample and the first and second reagents are individually supplied to the detecting part.

2. The cassette according to claim 1, wherein the first reagent is a washing reagent and the second reagent is a solution containing a substance which is bound to a hybridization-reacted nucleic acid to produce an electro-chemical reaction.

3. The cassette according to claim 1, wherein the first reagent reservoir is separated from the detecting part by air in the first channel.

4. The cassette according to claim 1, wherein
   the first channel includes a first horizontal channel part which is substantially extended in a horizontal direction in respect to a gravity direction and a first vertical channel part which is substantially extended in a vertical direction in respect to the gravity direction if the nucleic acid detection cassette is substantially arranged in a horizontal plane, the first horizontal and vertical channel parts have horizontal and vertical flowing channel cross-sections, the vertical flowing channel cross-section being smaller than the horizontal flowing channel cross-section, and
   the first reagent reservoir and the second reagent reservoir are formed mainly in the horizontal channel part.

5. The cassette according to claim 1, wherein a first pumping action is applied to the pump part in a first direction to supply the sample to the detecting part, and a second pumping action is applied to the pump part in a second direction opposite to the first direction to supply the first and second reagent to the detecting part.

6. The cassette according to claim 1, wherein a nucleic acid is amplified in the sample chamber.

7. The cassette according to claim 1, further comprising:

   a second injection port different from the first injection port, which allows a sample containing a target nucleic acid to be injected therethrough; and
   a second sample chamber defined in the second channel and communicated to the second injection port, the second sample chamber receiving the injected sample.

8. The cassette according to claim 1, at least one of the first channel, the second channel, and the detecting part comprises a liquid detection sensor which detects the supply of the sample, the first reagent, and the second reagent to the detecting part.

9. The cassette according to claim 7, further comprising:

   a sample storage chamber defined in the second channel between the sample chamber and the detecting part, which stores the first and second samples to be supplied from the sample chamber.

**10.** The cassette according to claim 9, wherein a third reagent which is a buffer solution is stored in the sample storage chamber.

**11.** The cassette according to claim 10, wherein

the sample storage chamber comprises channel ports, each of which is communicated to the sample chamber and the detecting part, and

the third reagent is so stored in the sample storage chamber as to keep a liquid level of the third reagent which is lower than the channel ports when the nucleic acid detection cassette is kept substantially horizontally,.

**12.** The cassette according to claim 11, wherein the sample storage chamber has a configuration in which a liquid level of the third reagent is lower than the channel ports when the sample is supplied in the third reagent.

**13.** A nucleic acid detection cassette, comprising:

a first injection port which allows a sample containing a target nucleic acid to be injected therethrough;

a first channel;

a second channel which is communicated to the first channel and the first injection port;

a detecting part comprising a detection chamber which is communicated to the first and second channels and is so formed as to be separated from the outside of the nucleic acid detection cassette, and a nucleic acid detection unit which identifies the target nucleic acid with the sample supplied to the detection chamber, the detecting part being arranged between the first and second channels;

a first reagent reservoir which is defined in the first channel and stores a first reagent while separating the first reagent from the sample via a gaseous body;

a sample chamber which is defined in the second channel and is communicated to the first injection port, the sample chamber receiving the injected sample;

a sample storage chamber which is defined in the second channel, comprises a first channel port communicated to the sample chamber and a second channel port communicated to the detecting part, and stores a sample liquid containing the sample supplied from the sample chamber, the first and second channel ports being opened in the sample storage chamber above a liquid level of the sample liquid; and

a pump part which is arranged between the first and second channels so as to be communicated to the first and second channels, the pump part forming a closed path together with the first channel, the second channel, and the detecting part, a pressure being applied to the pump part from the outside of the nucleic acid detection cassette so that the sample and the first reagent are individually supplied to the detecting part.

**14.** The cassette according to claim 13, wherein a nucleic acid is amplified in the sample chamber.

**15.** The cassette according to claim 13, wherein the first reagent reservoir is separated from the detecting part by a gaseous body in the first channel.

**16.** The cassette according to claim 13, wherein

a first pumping action is applied to the pump part in a first direction to supply a sample liquid containing the sample from the sample chamber into the sample storage chamber, and

a second pumping action is applied to the pump part in a second direction opposite to the first direction to supply the first reagent to the detecting part.

**17.** The cassette according to claim 13, comprising:

a second reagent reservoir defined in the first channel and stores a second reagent while separating the second reagent from the first reagent and the sample via a gaseous body, wherein the pump part supplies the second reagent to the detecting part.

**18.** The cassette according to claim 17, wherein the pump part supplies the second reagent to the detecting part due to the second pumping action in the second direction.

**19.** The cassette according to claim 17, wherein the first reagent includes a washing reagent and the second reagent includes a solution containing a substance which is bound to a hybridization-reacted nucleic acid to produce an electro-chemical reaction.

20. A nucleic acid detection system in which the cassette according to claim 12 is incorporated, comprising:

   a first mechanism which allows the second channel port to be communicated to the sample liquid; and
   a second mechanism configured to allow the sample liquid to be supplied to the detecting part via the second channel port.

21. The system according to claim 20, wherein the second mechanism comprises a pump operating unit which operates the pump part to supply the sample liquid to the detecting part.

22. A nucleic acid detection cassette, comprising:

   an injection port which allows a sample containing a target nucleic acid to be injected therethrough;
   a first channel;
   a second channel which is communicated to the first channel and the injection port;
   a detecting part comprising a detection chamber which is communicated to the first and second channels and is so formed as to be separated from the outside of the nucleic acid detection cassette, and a nucleic acid detection unit which detects an electric signal to identify the target nucleic acid with the sample supplied to the detection chamber, the detecting part being arranged between the first and second channels;
   a first reagent reservoir which is defined in the first channel and stores a first reagent while separating the first reagent from the sample via a gaseous body;
   a second reagent reservoir which is defined in the first channel and stores a second reagent while separating the second reagent from the first reagent and the sample via a gaseous body;
   a sample chamber which is defined in the second channel and is communicated to the injection port, the sample chamber receiving the injected sample;
   a fourth reagent reservoir which is communicated to the sample chamber and stores a fourth reagent to be supplied to the sample chamber;
   a sample storage chamber to store a sample liquid which corresponds to a mixture of the sample and the fourth reagent, the mixture being supplied from the sample chamber, the sample storage chamber being defined in the second channel and comprising a first channel port communicated to the sample chamber and a second channel port communicated to the detecting part;
   a first pump part provided on the cassette so as to be communicated to the second channel, the first pump part being actuated from the outside of the nucleic acid detection cassette so that the fourth reagent is supplied to the sample chamber; and
   a second pump part which is arranged between the first and second channels so as to be communicated to the first and second channels, the second pump part forming a closed path together with the first and second channels and the detection part, a pressure being applied from the outside of the nucleic acid detection cassette to so that the mixture of the sample and the fourth reagent and the first and second reagents individually are supplied to the detection part.

23. The cassette according to claim 22, wherein
   the first pump part includes a press pumping mechanism which comprises a pressing part having a space covered with an elastic member and which feeds the fourth reagent by pressing the pressing part from the outside of the nucleic acid detection cassette, and
   the following formula is satisfied:

$$(C + D) \leq A \leq 5 \times B.$$

   where A denotes an inner volume of the space of the press pumping mechanism; B denotes a channel volume between the space and the fourth reagent in the press pumping mechanism; C denotes a volume of the fourth reagent; and D denotes a channel volume between the fourth reagent and the sample chamber.

24. The cassette according to claim 22, wherein the nucleic acid is amplified in the sample chamber.

25. The cassette according to claim 22, wherein the first reagent is a washing reagent,
   the second reagent is a solution containing a substance which is bound to a hybridization-reacted nucleic acid to produce an electro-chemical reaction, and

the fourth reagent is a solution containing an enzyme.

26. The cassette according to claim 22, wherein the first reagent reservoir is separated from the detecting part by air in the first channel.

27. The cassette according to claim 22, wherein
a first pumping action is applied to the pump part in a first direction to supply a sample liquid containing the sample from the sample chamber into the sample storage chamber, and a second pumping action is applied to the pump part in a second direction opposite to the first direction to supply the first reagent to the detecting part.

28. A nucleic acid detection cassette, comprising:

a first plate unit provided with a first channel, first and second reagent reservoir defined in the first channel, and first and second reagents stored in the first and second reagent reservoirs, the first and second reagents being separated by a gaseous body;
a second plate unit provided with a second channel, an injection port which allows a sample containing a target nucleic acid to be injected through the injection port, and a sample chamber which is communicated to the injection port and is defined in the second channel, the sample chamber receiving the sample; and
a third plate unit provided with a detection chamber which is communicated to the first and second channels and is formed as to be separated from the outside of the nucleic acid detection cassette, and a nucleic acid detection unit which detects an electric signal to identify the target nucleic acid with the sample supplied to the detection chamber, the third plate unit further being provided with a pump part which is arranged between the first and second channels so as to be communicated to the first and second channels, the pump part forming a closed path together with the first and second channels and the nucleic acid detection unit, a pressure being applied to the pump part from the outside of the nucleic acid detection cassette so that the sample and the first and second reagents are individually supplied to the nucleic acid detection unit, wherein the first and second plate units are fixedly arranged to the opposite sides of the third plate unit.

29. The cassette according to claim 28, wherein the first plate unit comprises
a first substrate plate having one and the other surfaces on which a groove or a hole is formed;
a second substrate plate which is fixed on the one surface of the first substrate and closes the groove or the hole; and
a third substrate plate fixed on the other surface of the first substrate, which closes the groove or the hole to define the first channel.

30. The cassette according to claim 28, wherein the second plate unit includes an elastic member and the capacity of the sample storage chamber is variable by the external pressure.

31. The cassette according to claim 28, wherein
the second plate unit is further provided with a fourth reagent reservoir which is communicated to the sample chamber and stores a fourth reagent to be supplied to the sample chamber, and
the fourth reagent reservoir is covered by an elastic member, and has a variable capacity that is adjusted from the outside.

32. The cassette according to claim 28, wherein the first plate unit is further provided with a buffer cavity part communicated to the first channel to absorb a pressure increase in the cassette.

FIG. 1

F I G. 2A

F I G. 2B

F I G. 2C

CH1                                    BW

Reagent B          Reagent A

10

DNA chip
20
8
Detecting
part

Input sample   Input sample

12-1                              12-2
2-2

2-1                                CH2
18

Heating    Heating        FW
part       part

14-1       14-2

F I G. 3

16                 18                 20

Sample          Sample              Detecting
chamber         storage chamber     part

22                 24

F I G. 4A

16                 18                 20

Sample          Sample              Detecting
chamber         storage chamber     part

22                 24

F I G. 4B

FIG.5

(a)

16

Sample
chamber

18

Sample
storage chamber

20

Detecting
part

22

24

(b)

16

Sample
chamber

22

Sample
storage chamber

18

Rotation
(Indination)

24

20

Detecting
part

F I G. 6

FIG.7

(a)

16 Sample chamber

2-1  2-2

22

18 Sample storage chamber

24

20 Detecting part

(b)

16 Sample chamber

22

18 Sample storage chamber

24

20 Detecting part

(c)

16 Sample chamber

22

18 Sample storage chamber

24

20 Detecting part

EP 1 997 888 A1

16

Sample
chamber

22

Sample
storage chamber

24

18

30

20

Detecting
part

F I G. 8

16

Sample
chamber

22

32

24

18

20

Detecting
part

F I G. 9

16

22

24

20

Sample
chamber

Detecting
part

Sample
storage chamber

18

18A

F I G. 10A

16

22

24

20

Sample
chamber

Detecting
part

18

18A

32

F I G. 10B

16

22

Sample
chamber

36

34

38

20

Detecting
part

24

18

FIG. 11A

16

22

Sample
chamber

38

20

Detecting
part

24

34

18

FIG. 11B

F I G. 12

(c)

(a)

(b)

(d)

FIG. 13

Push mechanism — 92C

Push mechanism — 92B

Heating part for pretreatment — 14

Push mechanism — 92A

Heater for detecting part — 98

Pressure roller — 94

Connector for electrical contact — 92

CH12

10

10A

10B

74

18

10C

48B

48A

90B

90A

72

46

46

FIG. 14A

Input sample    Input sample

FIG. 14B

EP 1 997 888 A1

(a)          (b)          (c)          (d)

60           60           60           60

62           62           62           62

64           64           64           64

66           66           66           66

68           68           70           70
                          68           68

⊕—68         ⊕—68         ⊕—68         ⊕—68

(e)          (f)          (g)          (h)

FIG. 15

F I G.16

F I G. 17

F I G. 18

Inject sample into sample chamber — S10

↓

Temperature control (amplification processing) — S12

↓

Liquid feeding operation
(sample chamber → sample storage chamber) — S14

↓

Liquid feeding operation
(sample storage chamber → detecting part) — S16

↓

Temperature control (hybridization) — S18

↓

Liquid feeding operation
(cleaning agent → chip substrate) — S20

↓

Temperature control (cleaning) — S22

↓

Liquid feeding operation
(intercalating agent → detecting part) — S24

↓

Temperature control (intercalating agent reaction) — S26

↓

Electrochemical measurement — S28

F I G. 19

| **INTERNATIONAL SEARCH REPORT** | International application No. |
|---|---|
| | PCT/JP2007/056097 |

**A. CLASSIFICATION OF SUBJECT MATTER**
*C12N15/09*(2006.01)i, *C12M1/00*(2006.01)i, *C12Q1/68*(2006.01)i, *G01N27/28*(2006.01)i, *G01N27/327*(2006.01)i, *G01N35/08*(2006.01)i, *G01N37/00*(2006.01)i

According to International Patent Classification (IPC) or to both national classification and IPC

**B. FIELDS SEARCHED**

Minimum documentation searched (classification system followed by classification symbols)
C12N15/09, C12M1/00, C12Q1/68, G01N27/28, G01N27/327, G01N35/08, G01N37/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS/MEDLINE/WPIDS(STN), CAplus(STN), JMEDPlus(JDream2), JST7580(JDream2), JSTPlus(JDream2)

**C. DOCUMENTS CONSIDERED TO BE RELEVANT**

| Category* | Citation of document, with indication, where appropriate, of the relevant passages | Relevant to claim No. |
|---|---|---|
| A | JP 2005-261298 A  (Toshiba Corp.), 29 September, 2005 (29.09.05), & US 2006/0216812 A1 | 1-32 |
| A | JP 2005-323519 A  (Konica Minolta Sensing, Inc.), 24 November, 2005 (24.11.05), & US 2005/0255007 A1 | 1-32 |

☐ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22 June, 2007 (22.06.07) | 03 July, 2007 (03.07.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3007571 A
- JP 2005261298 A
- US 6830936 B
- US 6605454 B
- US P5776672 A
- US P5972692 A
- JP 2573443 B
- JP 2004012577 A